# EUROPEAN PATENT SPECIFICATION

(11) **EP 3 327 012 B1**
(45) Date of publication and mention of the grant of the patent: **17.03.2021**
(21) Application number: 16829901.4
(22) Date of filing: 22.07.2016
(51) Int. Cl.: C07D 401/04, C07B 63/00, A61K 31/454, A61P 37/08, A61P 5/04

(54) **CRYSTALLINE FORMS OF BILASTINE AND PREPARATION METHODS THEREOF**
KRISTALLINE FORMEN VON BILASTIN UND HERSTELLUNGSVERFAHREN DAFÜR
FORMES CRISTALLINES DE LA BILASTINE ET PROCÉDÉS POUR LEUR PRÉPARATION

(30) Priority: 24.07.2015 ES 201531103
(43) Date of publication of application: 30.05.2018
(73) Proprietor: URQUIMA, S.A., 08184 Palau-solità i Plegamans Barcelona (ES); Disproquima, S.A., 08227 Terrassa - Barcelona (ES)
(72) Inventor: DEL RÍO PERICACHO, José Luis, 08225 Terrassa (ES); PUIGJANER VALLET, Maria Cristina, 08029 Barcelona (ES); PROHENS LÓPEZ, Rafael, 08450 Llinars Del Vallès (ES); ARREDONDO MARTÍNEZ, Yolanda Esther, 08291 Ripollet (ES)
(74) Representative: ZBM Patents - Zea, Barlocci & Markvardsen
(86) International application number: PCT/ES2016/070560
(87) International publication number: WO 2017/017301

(56) References cited:
- EP-A1- 1 505 066
- WO-A1-03/089425
- WO-A2-2014/026657
- WO-A2-2014/026657
- CN-A- 104 447 683
- CN-A- 104 447 683
- SK-U1- 50 032 014
- SK-U1- 50 032 014
- BYRN ET AL.: "Pharmaceutical Solids: A strategic Approach to Regulatory Considerations", PHARMACEUTICAL RESEARCH, vol. 12, no. 7, 1 January 1995 (1995-01-01), pages 945-954, XP055399407, US ISSN: 0724-8741, DOI: 10.1023/A:1016241927429
- EPODOC and WPI abstracts of CN104447683 A, 2 pages (D4b).
- English machine translation of CN104447683 A, 4 pages (D4c).
- Seufert, G., Appeal number T2007/11-3.3.01, EPO Form 3350, Communication of the Board of Appeal pursuant to Article 15(1) of the Rules of Procedure of the Boards of Appeal, dated 10.07.2014.
- SUÑÉ-NEGRE ET AL.: INTERNATIONAL JOURNAL OF PHARMACEUTICS, vol. 470, 2014, pages 15-27,

## Description

### Technical Field

The present invention relates to a novel crystalline form of 4-[2-[4-[1-(2-ethoxyethyl)-1H-benzimidazol-2-yl]-1-piperidinyl)ethyl]-α,α-dimethyl-benzeneacetic acid (bilastine), preparation methods thereof, pharmaceutical compositions containing the same and the use thereof in the treatment of histamine-mediated disease processes and allergic reactions.

### Background art

Bilastine is the international common name of 4-[2-[4-[1-(2-ethoxyethyl)-1h-benzimidazol-2-yl]-1-piperidinyl)ethyl]-a,a-dimethyl-benzeneacetic acid, whose structure corresponds to the compound of formula (I):

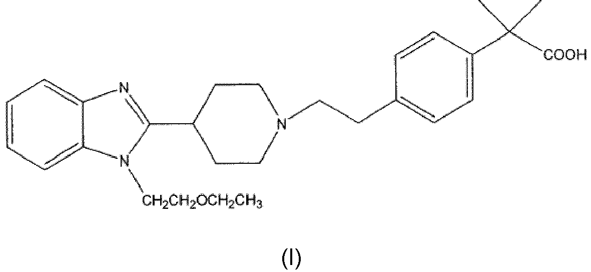

Bilastine is a selective H1 receptor antagonist, meaning that it is useful for treating histamine-mediated disease processes and allergic reactions, and especially for treating rhinoconjunctivitis and urticaria.

Bilastine as a product *per se,* as well as the preparation and use thereof as an H1 receptor antagonist has been described in the European patent EP0818454B1.

Subsequently, the European patent EP1505066B1 describes three crystalline forms of bilastine. Specifically, it describes the crystalline forms 1, 2 and 3 of bilastine, which are characterized by the infrared absorption spectrum and crystallographic parameters in the case of form 1. Furthermore, in the patent EP1505066B1, preparation methods of crystalline form 1 from a mixture of crystalline forms 2 and 3 are described. However, crystalline forms 2 and 3 of bilastine are easily converted into crystalline form 1. Patent document SK 7066 Y1 discloses crystalline dihydrate forms A and B of bilastine. Patent document CN104447683 discloses a monohydrate crystalline form of bilastine.

The different solid forms of a pharmaceutical active ingredient may have different characteristics and offer certain advantages, for example, with respect to stability, bioavailability, ease of the formulation and ease of administration, among others. Since some solid forms are more suitable for one type of formulation, and other forms for other different formulations, the development of novel solid forms makes it possible to improve the characteristics of pharmaceutical formulations that comprise them. Furthermore, depending on therapeutic indications, one or another pharmaceutical formulation may be preferable.

Especially desirable improvements to a novel crystalline form of bilastine include, for example, the improvement of physical and chemical properties, such as stability, solubility, fluidity, sedimentation rate, malleability or compressibility, for the purpose of facilitating the manufacture or formulation thereof; the improvement of pharmacokinetic properties, for the purpose of improving the release, absorption and/or bioavailability thereof, and the obtainment of more constant physical and chemical properties, since they make it possible to have greater ease and/or flexibility when formulated. For example, the improvement of the properties of dispersibility makes it possible to improve dispersion rates, especially if dispersed in a physiologically aqueous medium, while the reduction of hygroscopicity makes it possible to develop novel routes of administration; it is also desirable to obtain stable pharmaceutical compositions under several packaging and/or storage conditions. It is especially desirable that the novel solid forms of bilastine combine more than one or even most of the aforementioned advantages.

Therefore, there is a need to develop novel solid forms, in particular, novel crystalline forms of bilastine that are suitable for the use thereof in the pharmaceutical industry and, in particular, which make it easier to produce pharmaceutical compositions of bilastine in crystalline form that meet strict pharmaceutical standards.

### Description of the Invention

The inventors have found a novel crystalline form of bilastine that is stable under the preparation and storage conditions of the pharmaceutical composition, which ensures the reproducibility of the manufacture and quality of the composition. This novel crystalline form is henceforth called Eta crystalline form.

These properties of the novel crystalline form is especially advantageous in the case of bilastine, since most solid forms of bilastine have problems of stability under the normal preparation and storage conditions of the pharmaceutical compositions that contain them.

According to the data from the stability study of Table 1 of Example 9 of the present invention, the novel Eta form is more stable under normal storage conditions, and even under more extreme conditions, than the rest of the solid forms described by the inventors herein, and is even as stable as forms 1 and 2 and more stable than solid form 3 of bilastine described in the state of the art. As shown in Table 1, neither degradation products of bilastine nor the transformation of the Eta crystalline form into another crystalline form was detected.

The Eta crystalline form of the present invention not only has high stability, but also good physical and mechanical properties that allows it to be easily handled for the preparation of pharmaceutical compositions.

Furthermore, another advantage of the Eta crystalline form of the present invention lies in the fact that it is obtained through a reproducible and robust preparation method that obtains crystalline forms with high performance and elevated richness. Moreover, since the form is hydrated, said method requires water or mixtures of water with small amounts of organic solvents, meaning that it is environmentally friendly, easily industrializable and cost-effective.

An Alpha crystalline form of bilastine is disclosed having an X-ray diffractogram that comprises characteristic peaks at 8.7; 11.6; 13.4; 13.8, 14.0 and 17.7 ± 0.2 degrees 2 theta measured with an X-ray diffractometer with Cu Kα radiation (1.5418 Å).

The previously defined Alpha crystalline form is also characterized in that it further contains characteristic peaks at 18.6; 18.8; 20.1 and 21.1 ± 0.2 degrees 2 theta measured with an X-ray diffractometer with Cu Kα radiation (1.5418 Å). More specifically, the previously defined Alpha crystalline form is also characterized in that it has an X-ray diffractogram that further comprises peaks at 10.9; 12.2; 14.5; 15.0; 16.1; 17.4; 20.7; 21.4; 21.7; 21.9; 22.6; 23.3 and 23.5 ± 0.2 degrees 2 theta measured with an X-ray diffractometer with Cu Kα radiation (1.5418 Å).

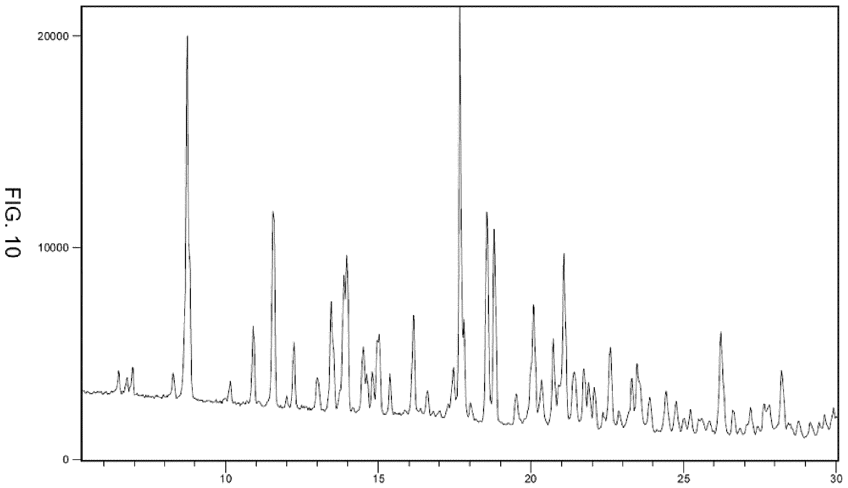

The previously defined Alpha crystalline form of bilastine has the X-ray diffractogram shown in FIG. 10. Said diffractogram differs from the diffractograms corresponding to other forms of bilastine known in the state of the art. The previously defined Alpha crystalline form of bilastine is characterized in that it shows the pattern of peaks, expressed in units of 2 theta degrees, 2θ (°), in the X-ray powder diffractogram shown in the following table:

| **2θ (°)** | **d(Å)** | **Intensity (%)** |
|---|---|---|
| 6.49 | 13.63 | 5.9 |
| 6.75 | 13.10 | 4.4 |
| 6.93 | 12.75 | 7.2 |
| 8.29 | 10.67 | 6.4 |
| 8.73 | 10.12 | 87.1 |
| 10.14 | 8.72 | 5.2 |
| 10.90 | 8.11 | 18.9 |
| 11.56 | 7.65 | 48.1 |
| 12.00 | 7.37 | 2.4 |
| 12.23 | 7.23 | 15.8 |
| 13.01 | 6.80 | 7.4 |
| 13.45 | 6.58 | 26.0 |
| 13.85 | 6.39 | 30.7 |
| 13.98 | 6.34 | 37.1 |
| 14.49 | 6.11 | 15.6 |
| 14.63 | 6.05 | 8.6 |
| 14.80 | 5.98 | 9.6 |
| 15.00 | 5.90 | 18.5 |
| 15.38 | 5.76 | 9.4 |
| 15.87 | 5.58 | 0.7 |
| 16.15 | 5.49 | 24.8 |
| 16.60 | 5.34 | 6.0 |
| 16.99 | 5.22 | 0.9 |
| 17.44 | 5.08 | 11.7 |
| 17.67 | 5.02 | 100 |
| 18.04 | 4.92 | 3.3 |
| 18.57 | 4.78 | 50.2 |
| 18.81 | 4.72 | 46.0 |
| 19.52 | 4.55 | 6.6 |
| 20.10 | 4.42 | 28.3 |
| 20.36 | 4.36 | 8.9 |
| 20.74 | 4.28 | 20.3 |
| 21.10 | 4.21 | 41.2 |
| 21.41 | 4.15 | 12.5 |
| 21.74 | 4.09 | 13.7 |
| 21.91 | 4.06 | 10.1 |
| 22.09 | 4.02 | 8.7 |
| 22.37 | 3.97 | 3.0 |
| 22.61 | 3.93 | 18.8 |
| 22.89 | 3.88 | 3.5 |
| 23.31 | 3.82 | 11.8 |
| 23.48 | 3.79 | 15.4 |
| 23.90 | 3.72 | 7.1 |
| 24.43 | 3.64 | 9.0 |
| 24.76 | 3.60 | 6.6 |
| 2501 | 3.56 | 2.7 |

The previously defined Alpha crystalline form is a hydrated form of bilastine.

The term "hydrated" refers to a compound that has water molecules that form part of the crystalline structure thereof. Furthermore, the hydrated crystalline form of the present invention may contain water molecules that do not form part of the crystalline structure. As described above, this crystalline form is especially advantageous because it is stable under the preparation and storage conditions of the pharmaceutical composition (cf. Example 9). Therefore, it is especially suitable for the preparation of a pharmaceutical composition of bilastine that meets strict pharmaceutical standards.

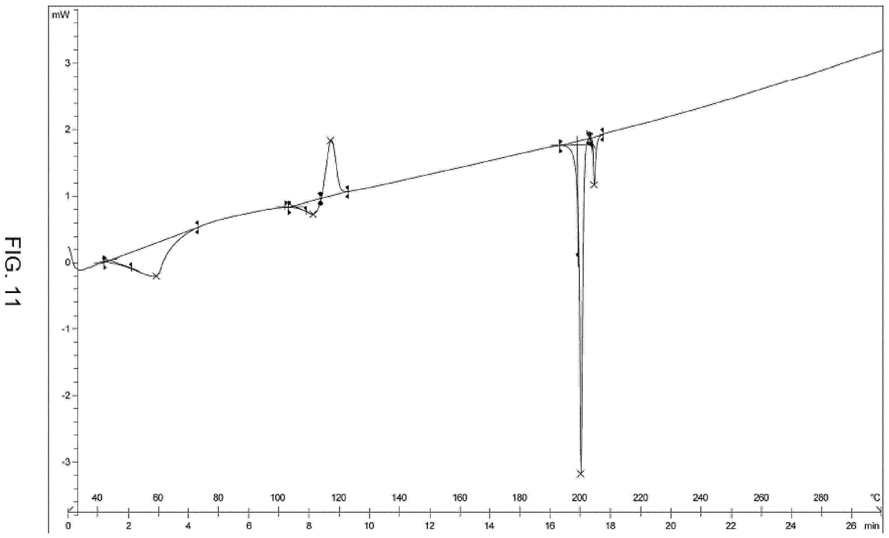

The previously defined Alpha crystalline form of bilastine is that which shows a first broad endothermic phenomenon with a peak at 59°C and an associated heat of 89.0 J/g; a second broad endothermic phenomenon with a peak at 111°C and an associated heat of 15.9 J/g followed by an exothermic phenomenon with a peak at 117°C and an associated heat of 43.5 J/g; a third endothermic phenomenon at 199°C with an associated heat of 100.4 J/g and a fourth endothermic phenomenon at 204°C with an associated heat of 9.7 J/g in the differential scanning calorimetry (DSC) diagram.The previously defined Alpha crystalline form of bilastine is that which has the DSC diagram shown in FIG. 11.

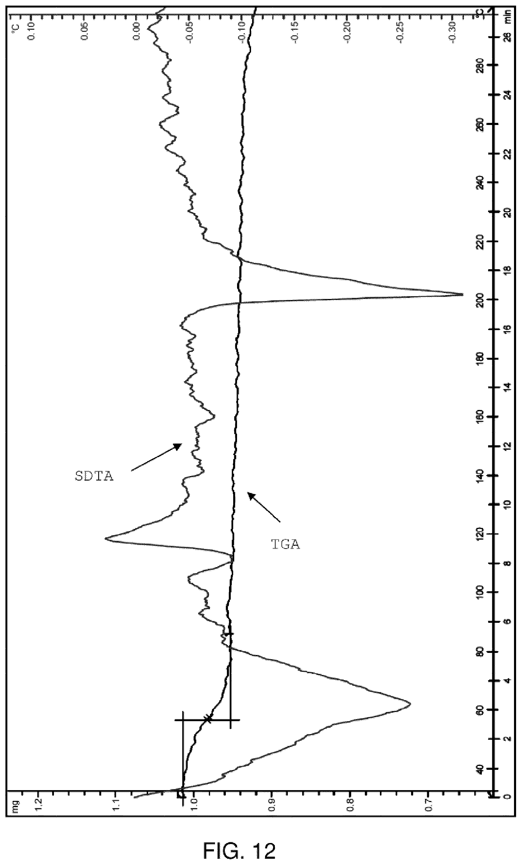

The previously defined Alpha crystalline form of bilastine is that which shows a weight loss of 5.9% from 30°C to 86°C in the diagram obtained by thermogravimetric analysis (TGA). This weight loss may be attributed to the water molecules present in the crystalline structure thereof. The previously defined Alpha crystalline form of bilastine is that which has the TGA diagram shown in FIG. 12.

A preparation method for the previously defined Alpha crystalline form of bilastine is also disclosed.

The preparation method for the previously defined Alpha crystalline form of bilastine is characterized in that it comprises a) obtaining the bilastine from a form 1 bilastine suspension in water.

The previously defined obtainment method for the Alpha crystalline form of bilastine (step a) is carried out with an amount of water comprised from 6 mL/g to 100 mL/g of the starting bilastine. The previously defined obtainment method for the Alpha crystalline form (step a) is carried out with an amount of water comprised from 20 mL/g to 100 mL/g of the starting bilastine.

The previously defined obtainment method for the Alpha form of bilastine (step a) comprises the following steps: a') heat a mixture of form 1 bilastine in water to a temperature comprised from 40°C to 95°C; a") cool the suspension obtained in step a') to room temperature; and a''') stir the suspension obtained in step a") during the time needed for the transformation to take place.

The previously defined obtainment method for the Alpha form of bilastine (step a) comprises the following steps: e') suspend form 1 bilastine in water at room temperature; and e") stir the suspension obtained in step e') during the period of time needed for the transformation to take place.

The term "room temperature" refers to a temperature comprised between 20°C and 25°C.

Step a") of the previously defined obtainment method for the Alpha form of bilastine may be carried out by using any cooling technique known in the state of the art, whether through contact with a cold bath or allowing it to cool by removing the heat source. The cooling step a") of the previously defined preparation method is carried out during a time comprised from 0.5 hours to 10 hours. The previously defined preparation method is characterized in that the step a") of the obtainment method for the Alpha form of bilastine is carried out at a temperature comprised from 20°C to 25°C.

Steps a''') and e") of the previously defined obtainment method for the Alpha form of bilastine may be carried out by using any stirring technique known in the state of the art. Steps a''') and e") of the previously defined obtainment method for the Alpha form of bilastine is carried out during a time comprised from 24 hours to 75 hours.

The previously defined obtainment method for the Alpha form of bilastine (step a) further comprises the following additional steps: b) isolate the crystalline bilastine obtained in step a); and c) separate the water from the bilastine obtained in step b).

The isolation of step b) and the separation of the solvent (water) of step c) of the previously defined obtainment method for the Alpha form of bilastine may be carried out by any conventional technique known in the state of the art. For example, the isolation of step b) may be carried out by filtration, while the separation of step c) may be carried out by subsequent drying. The steps of isolation b) and separation c) are generally carried out at a temperature comprised from 15°C to 25°C and during a time comprised from 30 minutes and 60 minutes. The drying time is shorter for higher temperatures. Drying is carried out at a temperature comprised from 20°C to 50°C, preferably under vacuum conditions.

In an alternative, the previously defined preparation method for the Alpha crystalline form is a method of crystallization that comprises the following steps: a1) dissolve bilastine in a mixture of water and ethanol at a temperature comprised from 75°C to 100°C, preferably at the reflux temperature of the solvent; a2) cool the solution obtained in step a1) to a temperature comprised from 50°C to 75°C; a3) seed the solution obtained in step a2) with the Alpha crystalline form and cool the resulting solution to a temperature comprised from 0°C to 25°C during the time needed for the crystallization to take place, preferably at a rate comprised from 0.2°C/min to 1°C/min; and a3') dry the crystalline form obtained in step a3) under reduced pressure at a temperature comprised from 25°C to 40°C until the water content is comprised from 6% to 8% by weight calculated by the Karl Fischer method, preferably until the water content is comprised of approximately 7% by weight calculated by the Karl Fischer method.

In an alternative, the previously defined preparation method for the Alpha crystalline form is a method of crystallization that comprises the following steps: a4) dissolve bilastine in a mixture of water, ice and a base at a temperature comprised from 15°C to 35°C; a5) add an aqueous acid solution to the solution obtained in step a4) until a pH comprised from 6 to 8 is reached for the crystallization to take place; and a6) dry the crystalline form obtained in step a5) under reduced pressure at a temperature comprised from 25°C to 40°C until the water content is comprised from 6% and 8% by weight calculated by the Karl Fischer method, preferably until the content is comprised of approximately 7% by weight calculated by the Karl Fischer method.

The previously defined Alpha crystalline form of bilastine has the X-ray diffractogram shown in FIG. 10, the DSC diagram shown in FIG. 11 and the TGA diagram shown in FIG. 12.

An aspect of the present invention relates to a hydrated crystalline form called Eta characterized in that it has an X-ray diffractogram that comprises characteristic peaks at 8.4; 9.6; 12.2; 13.2; 15.1; and 19.2 ± 0.2 degrees 2 theta measured with an X-ray diffractometer with Cu Kα radiation (1.5418 Å).

This Eta crystalline form is also characterized in that it further contains characteristic peaks at 19.7; 20.3; 21.5; and 23.4 ± 0.2 degrees 2 theta measured with an X-ray diffractometer with Cu Kα radiation (1.5418 Å). More specifically, this Eta crystalline form is also characterized in that it has an X-ray diffractogram that further comprises peaks at 14.0; 16.8; 17.5; 18.2 and 25.5 ± 0.2 degrees 2 theta measured with an X-ray diffractometer with Cu Kα radiation (1.5418 Å).

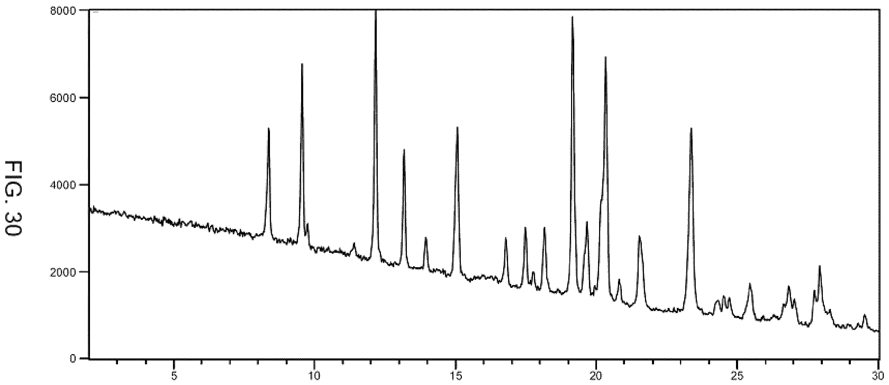

The Eta crystalline form of bilastine of the present invention has the X-ray diffractogram shown in FIG. 30. Said diffractogram differs from the diffractograms corresponding to other forms of bilastine known in the state of the art. This novel Eta crystalline form of bilastine is characterized in that it shows the pattern of peaks, expressed in units of 2 theta degrees, 2θ (°), in the X-ray powder diffractogram shown in the following table:

| **2θ (°)** | **d(Å)** | **Intensity (%)** |
|---|---|---|
| 8.37 | 10.56 | 41.0 |
| 9.55 | 9.26 | 64.9 |
| 9.74 | 9.08 | 7.8 |
| 11.40 | 7.76 | 4.3 |
| 12.18 | 7.27 | 89.7 |
| 13.19 | 6.71 | 42.1 |
| 13.95 | 6.35 | 11.2 |
| 15.07 | 5.88 | 52.7 |
| 16.79 | 5.28 | 16.0 |
| 17.49 | 5.07 | 21.0 |
| 17.77 | 4.99 | 5.8 |
| 18.18 | 4.88 | 22.6 |
| 19.18 | 4.63 | 100 |
| 19.67 | 4.51 | 26.7 |
| 20.16 | 4.40 | 34.0 |
| 20.34 | 4.37 | 83.9 |
| 20.83 | 4.26 | 7.4 |
| 21.52 | 4.13 | 25.3 |
| 23.35 | 3.81 | 66.8 |
| 24.26 | 3.67 | 4.9 |
| 24.51 | 3.63 | 7.0 |
| 24.73 | 3.60 | 6.4 |
| 25.46 | 3.50 | 11.1 |

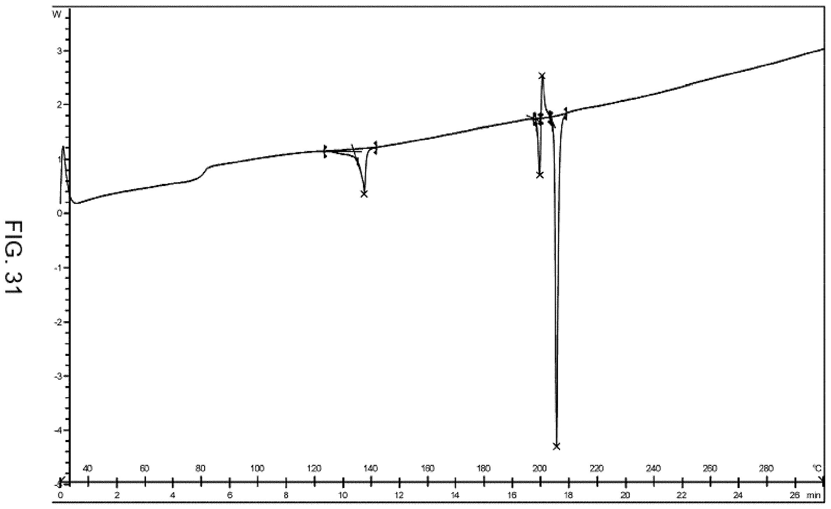

The previously defined Eta crystalline form of bilastine of the present invention shows a first broad endothermic phenomenon with a peak at 137°C and an associated heat of 35.4 J/g, followed by a second endothermic phenomenon at 198°C with an associated heat of 13.4 J/g overlapped with an exothermic phenomenon with a peak at 200°C and an associated heat of 14.0 J/g, and followed by a third endothermic phenomenon at 204°C with an associated heat of 101.3 J/g in the differential scanning calorimetry (DSC) diagram. Furthermore, the Eta crystalline form of bilastine of the present invention has the DSC diagram shown in FIG. 31.

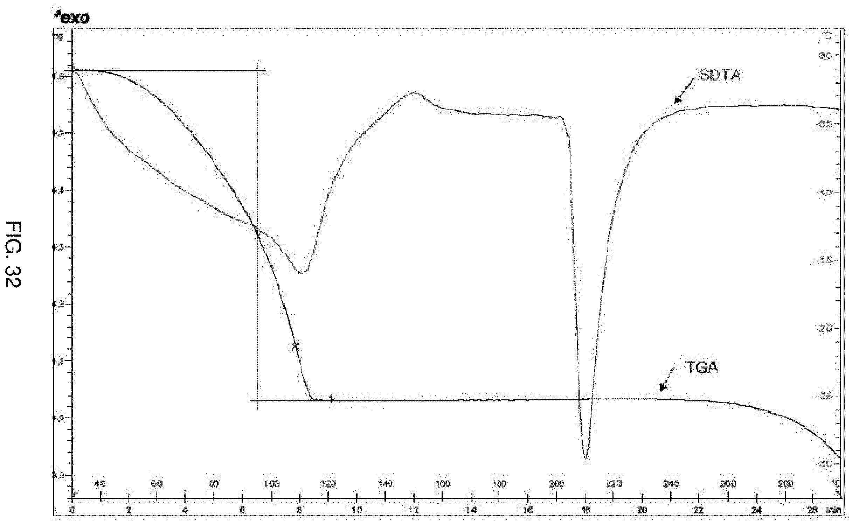

The previously defined Eta crystalline form of bilastine of the present invention shows a weight loss of 4.0% from 30°C to 120°C in the diagram obtained by thermogravimetric analysis (TGA). Furthermore, the Eta crystalline form of bilastine of the present invention has the TGA diagram shown in FIG. 32.

A second aspect of the present invention relates to a preparation method for the previously defined Eta crystalline form of bilastine of the present invention.

The previously defined Alpha crystalline form of bilastine may be transformed into the Eta crystalline form of bilastine of the present invention. Thus, in one embodiment of the invention, the obtainment method for the Eta form of bilastine is carried out based on the Alpha crystalline form, or alternatively, based on a mixture of the Alpha and Eta crystalline form that comprises dispersing the Alpha crystalline form in water, or alternatively, a mixture of the Alpha and Eta crystalline form during the time needed for the transformation into the Eta crystalline form of bilastine of said dispersion to take place. This method is advantageous because it makes it possible to obtain the Eta crystalline form of pure bilastine with high performance. In one embodiment, the time needed for the transformation into the Eta crystalline form of bilastine of said dispersion to take place is comprised from 1 day to 5 days, preferably from 3 days to 4 days.

Alternatively, form 1 of bilastine may be transformed into the Eta crystalline form of bilastine of the present invention. Therefore, in one embodiment of the invention, the obtainment method for the Eta form of bilastine is carried out based on crystalline form 1 which comprises dispersing crystalline form 1 in water during the time needed for the transformation into the Eta crystalline form of bilastine of said dispersion to take place. This method is also advantageous because it makes it possible to obtain the Eta crystalline form of pure bilastine with high performance. In one embodiment, the time needed for the transformation into the Eta crystalline form of bilastine of said dispersion to take place is more than 5 days.

The term "Eta crystalline form of pure bilastine" means no other crystalline form of bilastine is detectable by an X-ray powder diffractogram measured with an X-ray diffractometer with Cu-K radiationα λ=1.5406 Å.

In one embodiment of the invention, the method for crystallizing the Eta form of bilastine comprises the following steps: i) dissolve bilastine in a mixture of water and acetonitrile at a temperature comprised from 40°C to 70°C; ii) cool the solution obtained in step i) to a temperature comprised from 25°C to 50°C; iii) seed the solution obtained in step ii) with the Eta crystalline form and cool the resulting solution to a temperature comprised from 0°C to 30°C during the time needed for the crystallization to take place; and iv) dry the crystalline form obtained in step iii) under reduced pressure at a temperature comprised from 25°C to 40°C until the water content is comprised from 3.5% to 4% by weight calculated by the Karl Fischer method; the temperature is preferably comprised from 30°C to 35°C.

Alternatively, the Zeta form of bilastine defined in the present invention may be transformed into the Eta crystalline form of bilastine. Therefore, in one embodiment of the invention, the obtainment method for the Eta form of bilastine is carried out based on the Zeta crystalline form which comprises maintaining said crystalline form at a temperature comprised from 20°C to 50°C during the time needed for the transformation into the Eta crystalline form of bilastine to take place. In one embodiment, said transformation is carried out under reduced pressure at a temperature comprised from 25°C to 40°C during the time needed for the transformation into the Eta crystalline form of bilastine to take place.

In one particular embodiment, the obtainment method for the Eta form of bilastine is carried out based on the Zeta crystalline form which comprises maintaining said crystalline form under reduced pressure at a temperature comprised from 25°C to 40°C, preferably comprised from 30°C and 35°C until the water content is comprised from 3.5% to 4% by weight calculated by the Karl Fischer method.

In another particular embodiment, the obtainment method for the Eta form of bilastine is carried out based on the Zeta crystalline form which comprises maintaining said crystalline form at a temperature comprised from 35°C to 45°C and at a relative humidity comprised from 65% to 80% during the time needed for the transformation into the Eta crystalline form of bilastine to take place, preferably during a time longer than 2 weeks.

A third aspect of the invention relates to a pharmaceutical composition characterized in that it comprises a therapeutically effective amount of Eta crystalline form of bilastine of the present invention, along with suitable amounts of pharmaceutically acceptable excipients or carriers.

A "therapeutically effective amount" of Eta crystalline form of bilastine of the present invention relates to the amount of said Eta crystalline form that provides a therapeutic effect after the administration thereof.

The term "pharmaceutically acceptable" refers to the excipients or carriers suitable for use in pharmaceutical technology for the preparation of compositions for medical use. Said excipients or carriers must be pharmaceutically acceptable in the sense that they must be compatible with the rest of the ingredients of the pharmaceutical composition. They must also be suitable for use in contact with human or animal tissues or organs without showing excessive toxicity, irritation, allergic response, immunogenicity or other problems or complications relating to a reasonable risk/benefit ratio.

A fourth aspect of the present invention relates to the use of the previously defined Eta crystalline form of bilastine of the present invention for the manufacture of a medicament to treat histamine-mediated disease processes and allergic reactions, preferably to treat histamine-mediated disease processes and allergic reactions selected from the group consisting of the symptomatic treatment of seasonal allergic rhinoconjunctivitis, the symptomatic treatment of perennial allergic rhinoconjunctivitis and the treatment of urticaria.

The inventors have found other novel crystalline forms that are not known in the state of the art, which are herein disclosed.

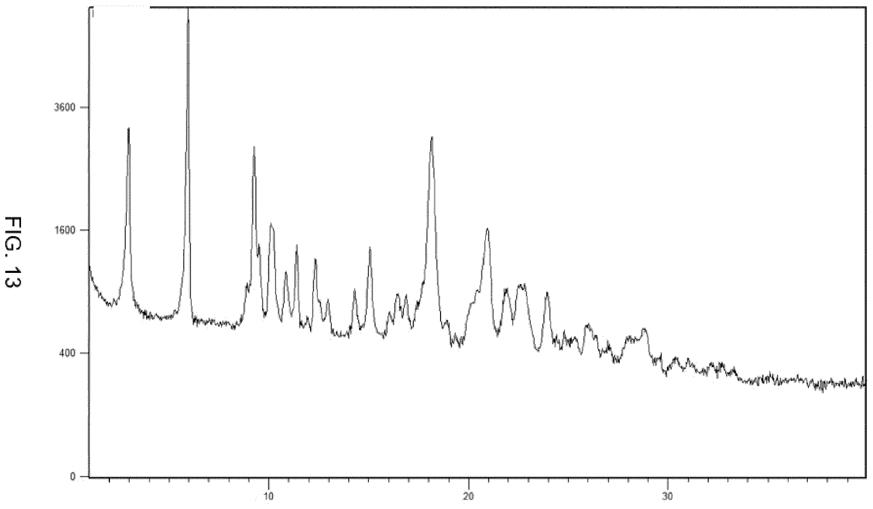

Thus, another novel crystalline form of bilastine is the crystalline form called the Beta form, which has the X-ray diffractogram shown in FIG. 13. Said diffractogram differs from the diffractograms corresponding to other forms of bilastine known in the state of the art. This novel Beta crystalline form of bilastine is characterized in that it shows the pattern of peaks, expressed in units of 2 theta degrees, 2θ (°), in the X-ray powder diffractogram shown in the following table:

| **2θ (°)** | **d(Å)** | **Intensity (%)** |
|---|---|---|
| 3.10 | 29.65 | 48.8 |
| 5.96 | 14.83 | 100 |
| 9.27 | 9.54 | 44.5 |
| 9.54 | 9.27 | 15.0 |
| 10.08 | 8.77 | 19.9 |
| 10.25 | 8.63 | 18.9 |
| 10.85 | 8.16 | 10.2 |
| 11.40 | 7.76 | 16.6 |
| 12.34 | 7.17 | 13.8 |
| 12.94 | 6.84 | 5.2 |
| 14.28 | 6.20 | 7.2 |
| 15.05 | 5.89 | 16.8 |
| 16.03 | 5.53 | 3.8 |
| 16.49 | 5.38 | 7.4 |
| 16.89 | 5.25 | 7.3 |
| 18.16 | 4.88 | 49.8 |
| 18.94 | 4.68 | 3.3 |
| 20.07 | 4.42 | 6.0 |
| 20.93 | 4.24 | 23 |
| 21.84 | 4.07 | 8.9 |

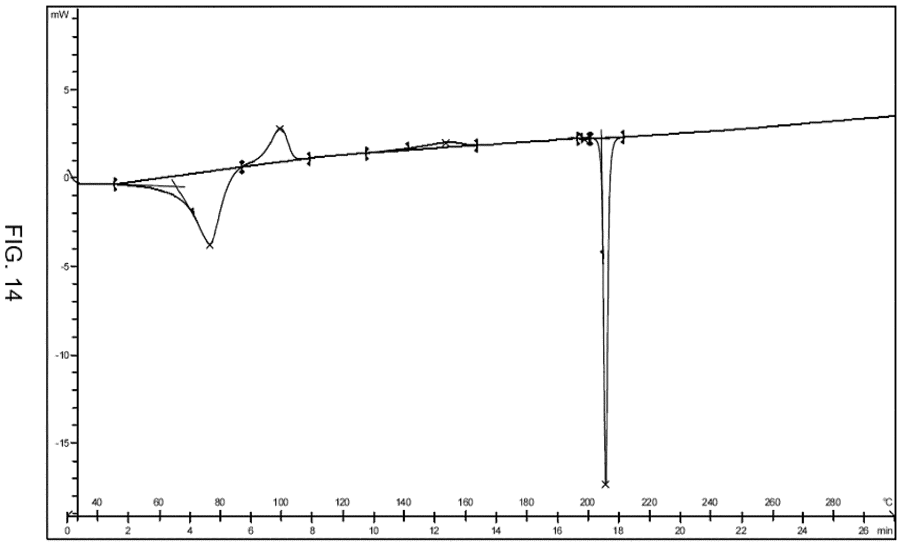

The previously defined Beta crystalline form of bilastine shows a first broad endothermic phenomenon with a peak at 76°C and an associated heat of 168.8 J/g, followed by an exothermic phenomenon with a peak at 99°C and an associated heat of 44.0 J/g in the differential scanning calorimetry (DSC) diagram. A broad exothermic phenomenon is also shown with a peak at 154°C and an associated heat of 13.5 J/g, followed by an endothermic phenomenon at 197°C with an associated heat of 0.3 J/g and another endothermic phenomenon at 204°C with an associated heat of 103.6 J/g. Furthermore, the Beta crystalline form of bilastine has the DSC diagram shown in FIG. 14.

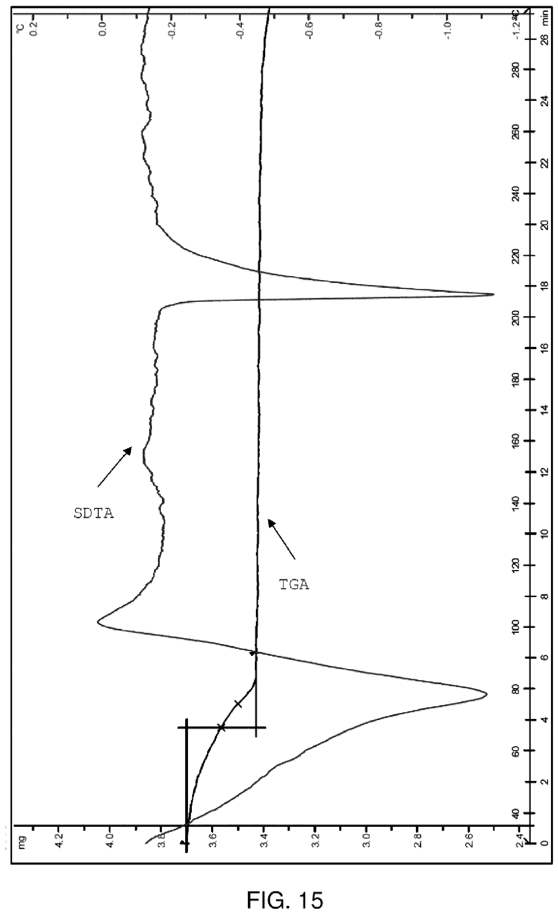

The previously defined Beta crystalline form of bilastine shows a weight loss of 7.4% from 30°C to 91°C in the diagram obtained by thermogravimetric analysis (TGA). Furthermore, the Beta crystalline form of bilastine has the TGA diagram shown in FIG. 15.

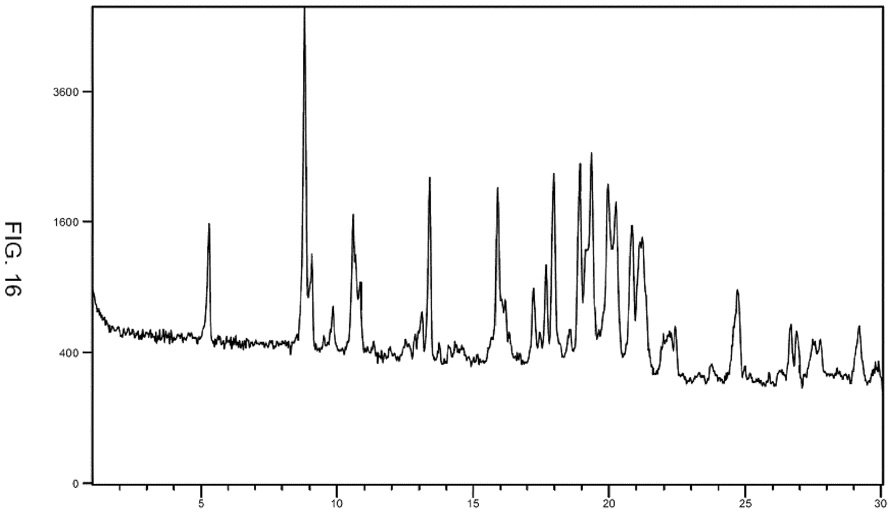

Another novel crystalline form of bilastine is called the Delta crystalline form, which has the X-ray diffractogram shown in FIG. 16. Said diffractogram differs from the diffractograms corresponding to other forms of bilastine known in the state of the art. This novel Delta crystalline form of bilastine is characterized in that it shows the pattern of peaks, expressed in units of 2 theta degrees, 2θ (°), in the X-ray powder diffractogram shown in the following table:

| **2θ (°)** | **d(Å)** | **Intensity (%)** |
|---|---|---|
| 5.28 | 16.75 | 22.5 |
| 8.82 | 10.03 | 100 |
| 9.07 | 9.75 | 16.4 |
| 9.85 | 8.98 | 6.4 |
| 10.57 | 8.37 | 24.8 |
| 10.85 | 8.15 | 11.3 |
| 12.55 | 7.05 | 1.5 |
| 13.10 | 6.76 | 6.4 |
| 13.38 | 6.61 | 37.3 |
| 14.53 | 6.10 | 1.2 |
| 15.88 | 5.58 | 34.8 |
| 17.23 | 5.15 | 11.5 |
| 17.68 | 5.02 | 16.2 |
| 17.96 | 4.94 | 39.6 |
| 18.54 | 4.78 | 4.9 |
| 18.92 | 4.69 | 43.0 |
| 19.37 | 4.58 | 46.1 |
| 19.95 | 4.45 | 36.6 |
| 20.25 | 4.38 | 31.7 |
| 20.84 | 4.26 | 26.5 |
| 21.09 | 4.21 | 21.3 |
| 21.23 | 4.18 | 22.6 |
| 21.98 | 4.04 | 4.1 |
| 22.45 | 3.96 | 6.1 |
| 23.75 | 3.74 | 1.5 |
| 24.74 | 3.60 | 12.3 |
| 26.65 | 3.34 | 7.0 |

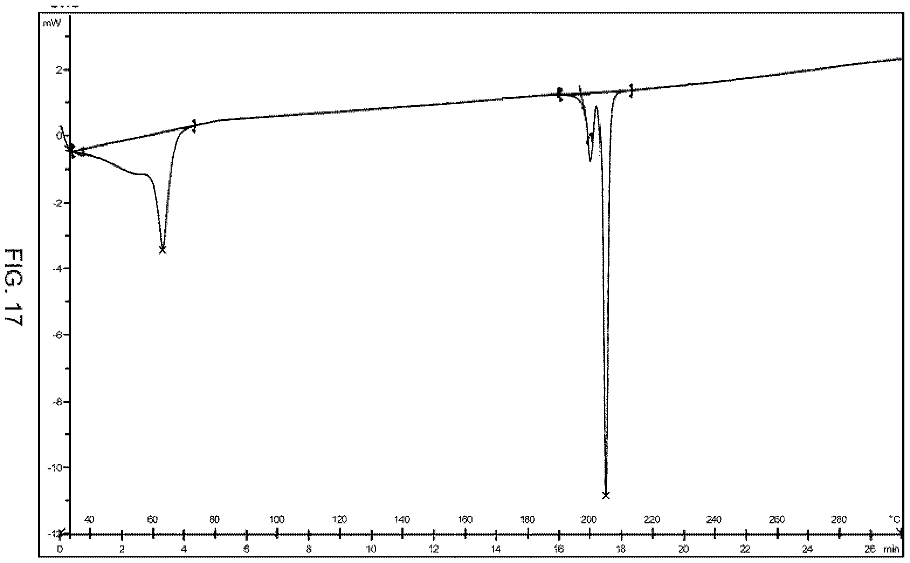

The previously defined Delta crystalline form of bilastine shows a first broad endothermic phenomenon with a peak at 63°C and an associated heat of 117.8 J/g, followed by two endothermic phenomena overlapped at 197°C with a total associated heat of 101.6 J/g in the differential scanning calorimetry (DSC) diagram. Furthermore, the Delta crystalline form of bilastine has the DSC diagram shown in FIG. 17.

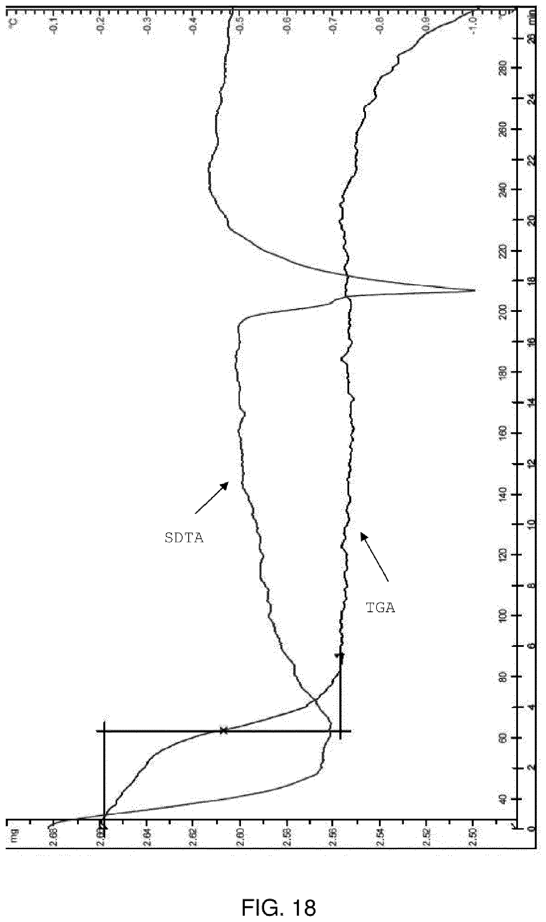

The previously defined Delta crystalline form of bilastine shows a weight loss of 3.8% from 30°C to 87°C in the diagram obtained by thermogravimetric analysis (TGA). Furthermore, the Delta crystalline form of bilastine has the TGA diagram shown in FIG. 18.

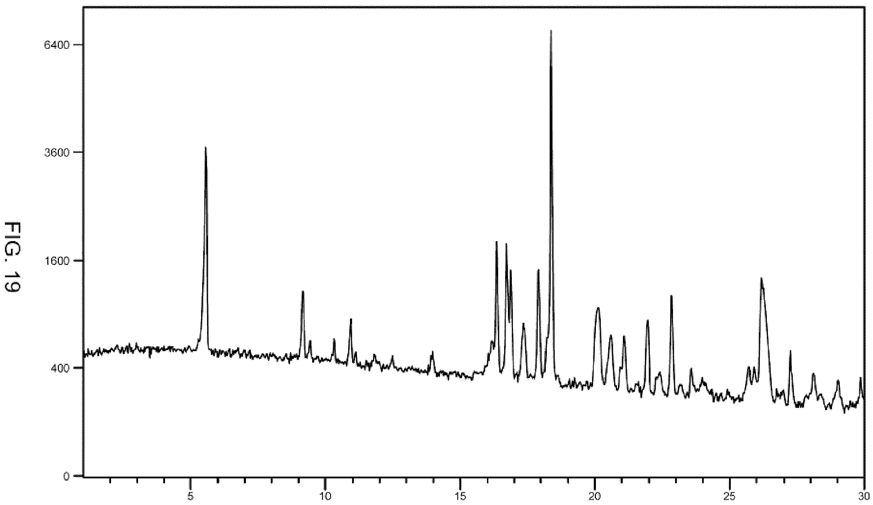

Another novel crystalline form of bilastine is called the Epsilon crystalline form, which has the X-ray diffractogram shown in FIG. 19. Said diffractogram differs from the diffractograms corresponding to other forms of bilastine known in the state of the art. This novel Epsilon crystalline form of bilastine is characterized in that it shows the pattern of peaks, expressed in units of 2 theta degrees, 2θ (°), in the X-ray powder diffractogram shown in the following table:

| **2θ (°)** | **d(Å)** | **Intensity (%)** |
|---|---|---|
| 5.56 | 15.88 | 49.1 |
| 9.16 | 9.65 | 11.1 |
| 9.42 | 9.39 | 2.8 |
| 10.31 | 8.58 | 3.3 |
| 10.92 | 8.10 | 6.4 |
| 11.11 | 7.96 | 1.7 |
| 11.82 | 7.49 | 1.5 |
| 12.47 | 7.10 | 1.6 |
| 13.95 | 6.35 | 2.4 |
| 16.18 | 5.48 | 4.5 |
| 16.36 | 5.42 | 24.0 |
| 16.73 | 5.30 | 23.2 |
| 16.89 | 5.25 | 15.7 |
| 17.38 | 5.10 | 6.5 |
| 17.91 | 4.95 | 18.4 |
| 18.38 | 4.83 | 100 |
| 20.16 | 4.40 | 9.6 |
| 20.59 | 4.31 | 6.3 |
| 21.08 | 4.21 | 6.4 |
| 21.55 | 4.12 | 0.6 |
| 21.94 | 4.05 | 8.9 |
| 22.42 | 3.97 | 1.9 |
| 22.85 | 3.89 | 13.7 |
| 23.17 | 3.84 | 0.7 |
| 23.57 | 3.77 | 2.7 |
| 24.01 | 3.71 | 1.3 |
| 25.69 | 3.47 | 3.2 |

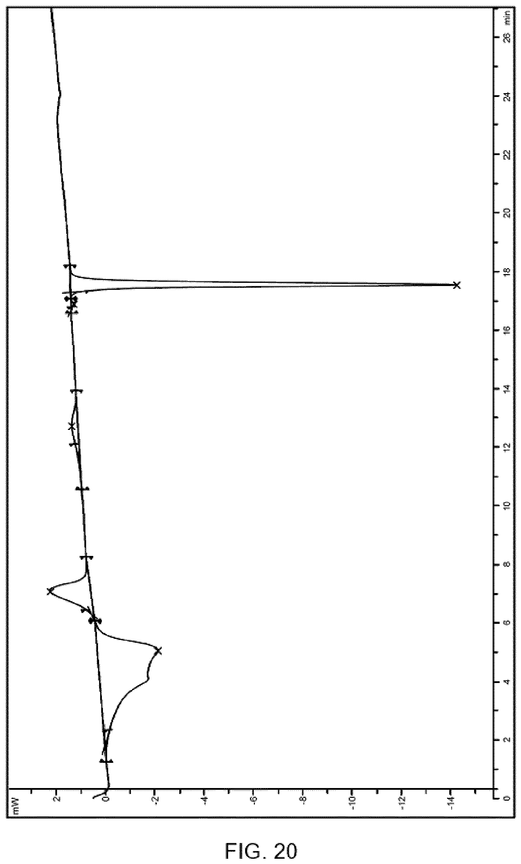

The previously defined Epsilon crystalline form of bilastine shows a first broad endothermic phenomenon with a peak at 81°C and an associated heat of 185.3 J/g, followed by an exothermic phenomenon with a peak at 101°C and an associated heat of 48.2 J/g in the differential scanning calorimetry (DSC) diagram. A broad exothermic phenomenon is also shown with a peak at 157°C and an associated heat of 14.2 J/g, followed by an endothermic phenomenon at 197°C with an associated heat of 0.9 J/g and another endothermic phenomenon at 203°C with an associated heat of 111.0 J/g. Furthermore, the Epsilon crystalline form of bilastine has the DSC diagram shown in FIG. 20.

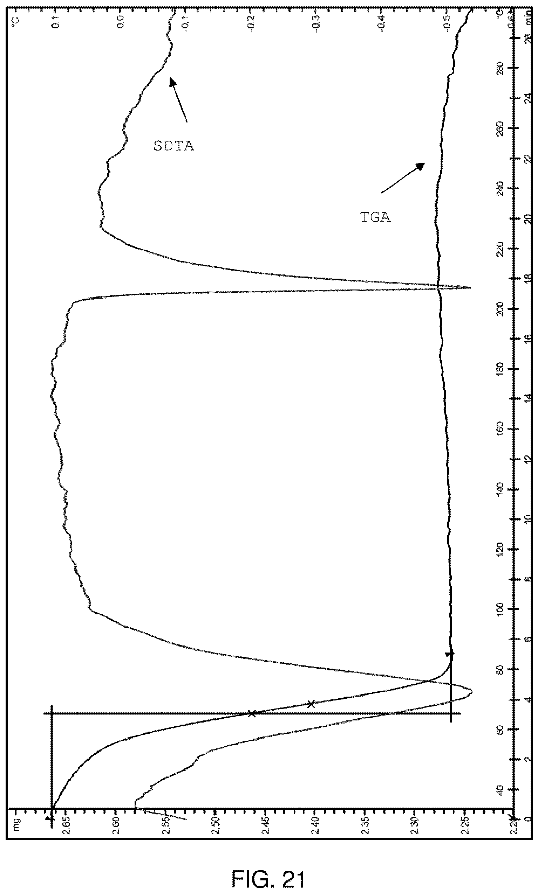

The previously defined Epsilon crystalline form of bilastine shows a weight loss of 15.0% from 30°C to 85°C in the diagram obtained by thermogravimetric analysis (TGA). Furthermore, the Epsilon crystalline form of bilastine has the TGA diagram shown in FIG. 21.

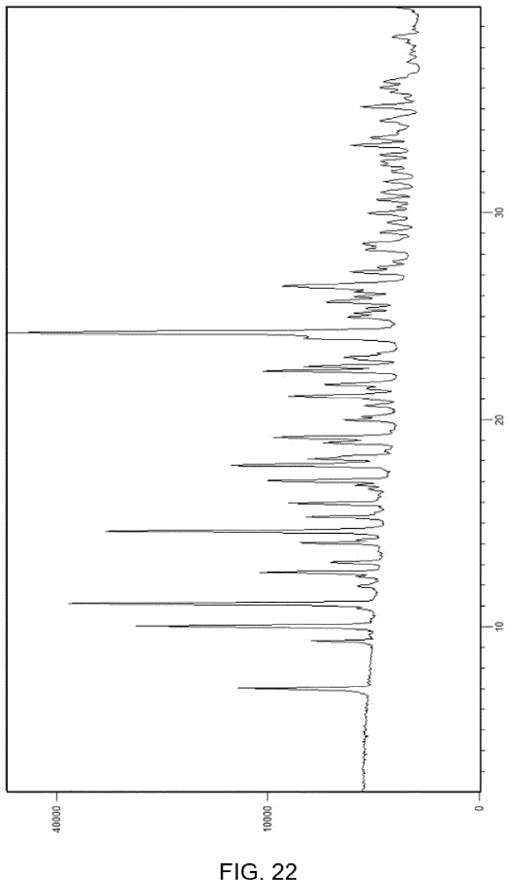

Another novel crystalline form of bilastine is called the Gamma A crystalline form, which has the X-ray diffractogram shown in FIG. 22. Said diffractogram differs from the diffractograms corresponding to other forms of bilastine known in the state of the art. This novel Gamma A crystalline form of bilastine is a chloroform solvate characterized in that it shows the pattern of peaks, expressed in units of 2 theta degrees, 2θ (°), in the X-ray powder diffractogram shown in the following table:

| **2θ (°)** | **d(Å)** | **Intensity (%)** |
|---|---|---|
| 7.00 | 12.62 | 20.6 |
| 9.31 | 9.49 | 7.5 |
| 10.03 | 8.82 | 50.0 |
| 11.12 | 7.95 | 74.1 |
| 11.96 | 7.40 | 1.9 |
| 12.63 | 7.01 | 17.1 |
| 13.10 | 6.76 | 5.4 |
| 14.04 | 6.31 | 10.3 |
| 14.21 | 6.23 | 2.2 |
| 14.61 | 6.06 | 60.8 |
| 15.30 | 5.79 | 9.1 |
| 15.96 | 5.55 | 12.4 |
| 16.64 | 5.33 | 1.5 |
| 16.84 | 5.27 | 2.9 |
| 17.06 | 5.20 | 16.7 |
| 17.79 | 4.98 | 23.8 |
| 18.09 | 4.90 | 9.4 |
| 18.24 | 4.86 | 4.2 |
| 18.49 | 4.80 | 0.1 |
| 18.88 | 4.70 | 7.2 |
| 19.15 | 4.64 | 15.3 |
| 19.97 | 4.45 | 4.6 |
| 20.15 | 4.41 | 2.4 |
| 20.67 | 4.30 | 2.3 |
| 21.12 | 4.21 | 12.9 |
| 21.48 | 4.14 | 2.2 |
| 21.69 | 4.10 | 7.3 |
| 22.36 | 3.98 | 18.0 |
| 22.58 | 3.94 | 10.5 |
| 22.91 | 3.88 | 3.7 |
| 23.04 | 3.86 | 5.0 |
| 23.92 | 3.72 | 10.2 |
| 24.21 | 3.68 | 100 |

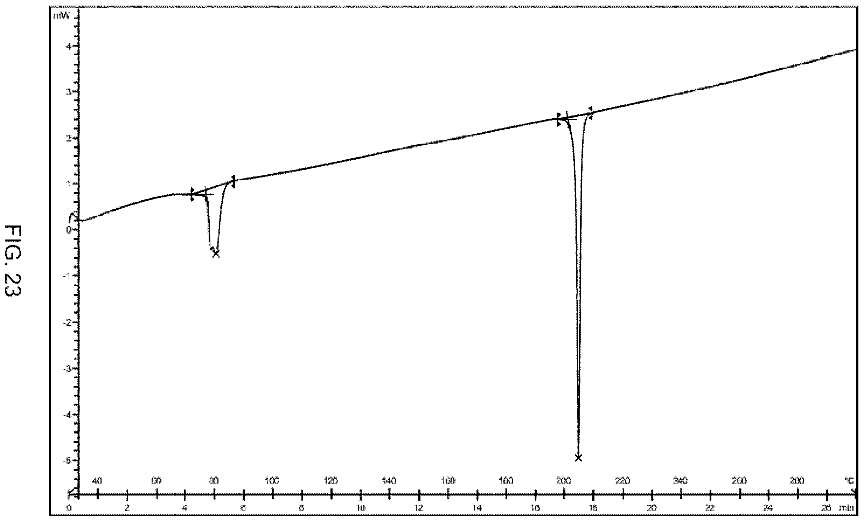

The previously defined Gamma A crystalline form of bilastine shows a first broad endothermic phenomenon with a peak at 80°C and an associated heat of 51.0 J/g followed by a second endothermic phenomenon at 201°C with an associated heat of 87.6 J/g in the differential scanning calorimetry (DSC) diagram. Furthermore, the Gamma A crystalline form of bilastine has the DSC diagram shown in FIG. 23.

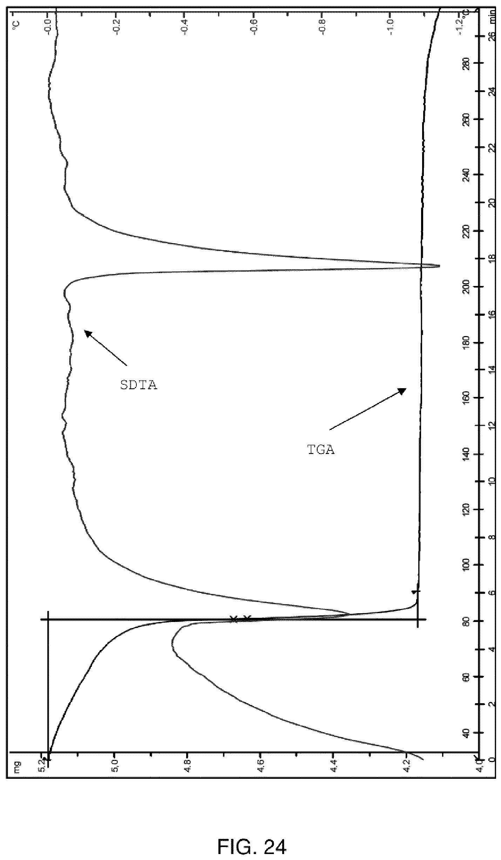

The previously defined Gamma A crystalline form of bilastine shows a weight loss of 19.0% from 29°C to 90°C in the diagram obtained by thermogravimetric analysis (TGA). Furthermore, the Gamma A crystalline form of bilastine has the TGA diagram shown in FIG. 24.

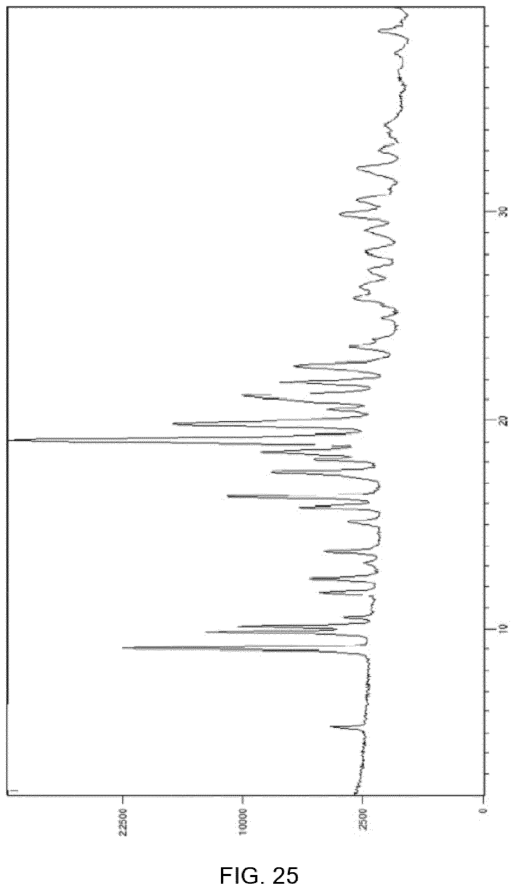

Another novel crystalline form of bilastine is called the Gamma B crystalline form, which has the X-ray diffractogram shown in FIG. 25. Said diffractogram differs from the diffractograms corresponding to other forms of bilastine known in the state of the art. This novel Gamma B crystalline form of bilastine is a chloroform solvate characterized in that it shows the pattern of peaks, expressed in units of 2 theta degrees, 2θ (°), in the X-ray powder diffractogram shown in the following table:

| **2θ (°)** | **d(Å)** | **Intensity (%)** |
|---|---|---|
| 5.28 | 16.74 | 4.3 |
| 9.04 | 9.79 | 53.8 |
| 9.86 | 8.97 | 30.0 |
| 10.13 | 8.73 | 22.2 |
| 10.58 | 8.36 | 3.3 |
| 11.74 | 7.54 | 6.8 |
| 12.39 | 7.14 | 8.4 |
| 13.12 | 6.75 | 1.2 |
| 13.68 | 6.47 | 6.5 |
| 15.13 | 5.86 | 3.4 |
| 15.84 | 5.59 | 10.7 |
| 16.33 | 5.43 | 25.7 |
| 17.55 | 5.05 | 16.2 |
| 18.13 | 4.89 | 8.6 |
| 18.50 | 4.80 | 18.4 |
| 19.07 | 4.65 | 100 |
| 19.80 | 4.48 | 40.4 |
| 20.52 | 4.33 | 7.0 |
| 20.90 | 4.25 | 10.4 |
| 21.16 | 4.20 | 22.3 |
| 21.86 | 4.06 | 15.2 |
| 22.68 | 3.92 | 12.4 |
| 23.56 | 3.77 | 4.2 |
| 23.93 | 3.72 | 1.8 |

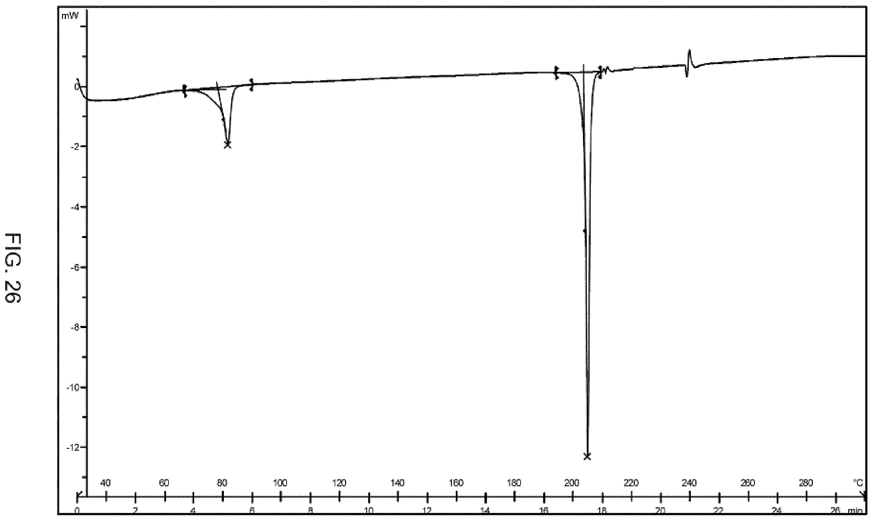

The previously defined Gamma B crystalline form of bilastine shows a first broad endothermic phenomenon with a peak at 82°C and an associated heat of 35.9 J/g, followed by a second endothermic phenomenon at 203°C with an associated heat of 95.1 J/g in the differential scanning calorimetry (DSC) diagram. Furthermore, the Gamma B crystalline form of bilastine has the DSC diagram shown in FIG. 26.

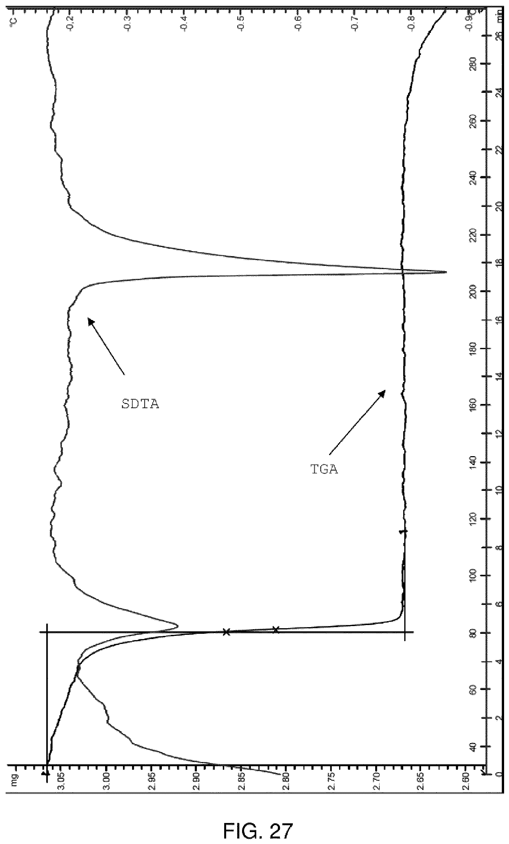

The previously defined Gamma B crystalline form of bilastine shows a weight loss of 13.0% from 29°C to 116°C in the diagram obtained by thermogravimetric analysis (TGA). Furthermore, the Gamma B crystalline form of bilastine has the TGA diagram shown in FIG. 27.

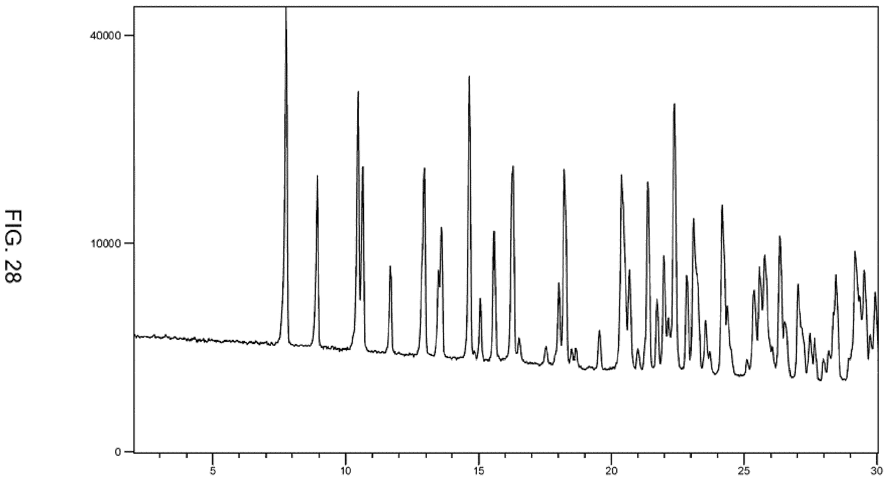

Another novel crystalline form of bilastine is called the Zeta crystalline form, which has the X-ray diffractogram shown in FIG. 28. Said diffractogram differs from the diffractograms corresponding to other forms of bilastine known in the state of the art. This novel Zeta crystalline form of bilastine is characterized in that it shows the pattern of peaks, expressed in units of 2 theta degrees, 2θ (°), in the X-ray powder diffractogram shown in the following table:

| **2θ (°)** | **d(Å)** | **Intensity (%)** |
|---|---|---|
| 7.76 | 11.39 | 100 |
| 8.93 | 9.90 | 34.5 |
| 10.45 | 8.46 | 64.3 |
| 10.63 | 8.32 | 40.0 |
| 11.69 | 7.57 | 13.0 |
| 12.96 | 6.83 | 38.3 |
| 13.60 | 6.51 | 21.7 |
| 14.65 | 6.05 | 69.9 |
| 15.07 | 5.88 | 7.9 |
| 15.58 | 5.69 | 21.6 |
| 16.28 | 5.44 | 39.3 |
| 16.53 | 5.36 | 2.5 |
| 17.54 | 5.06 | 1.5 |
| 18.04 | 4.92 | 11 |
| 18.25 | 4.86 | 37.4 |
| 18.61 | 4.77 | 0.6 |
| 19.54 | 4.54 | 3.7 |
| 20.38 | 4.36 | 36.8 |
| 20.69 | 4.29 | 13.3 |
| 21.01 | 4.23 | 1.7 |
| 21.37 | 4.16 | 34.9 |
| 21.73 | 4.09 | 8.5 |
| 21.99 | 4.04 | 16.8 |
| 22.37 | 3.97 | 60.9 |
| 22.85 | 3.89 | 12.4 |
| 23.10 | 3.85 | 25.1 |
| 23.25 | 3.83 | 11.2 |
| 23.54 | 3.78 | 5.6 |
| 24.18 | 3.68 | 29.1 |
| 24.37 | 3.65 | 6.9 |
| 25.12 | 3.54 | 1.1 |

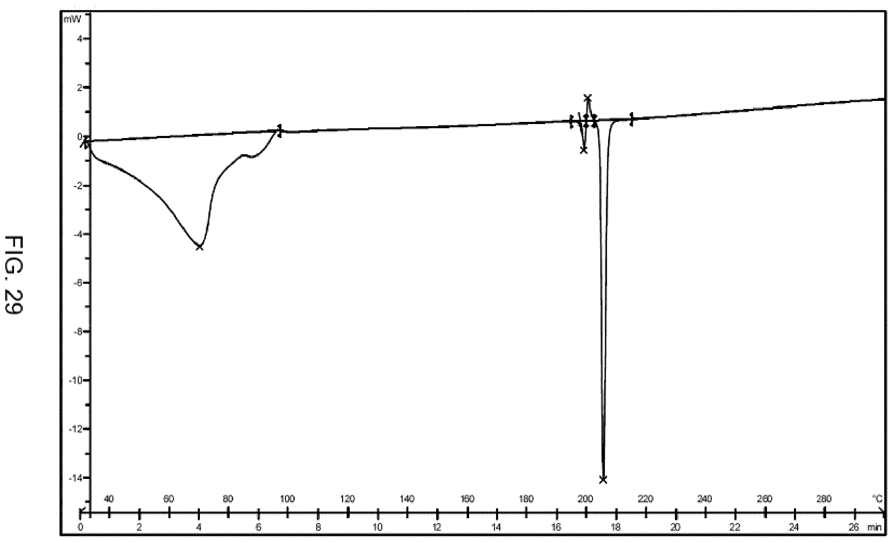

The previously defined Zeta crystalline form of bilastine shows a first broad endothermic phenomenon with a peak at 70°C and an associated heat of 506.5 J/g. a second endothermic phenomenon at 198°C with an associated heat of 5.6 J/g overlapped with an exothermic phenomenon with a peak at 201°C and an associated heat of 4.6 J/g, followed by a third endothermic phenomenon with an associated heat of 100.2 J/g in the differential scanning calorimetry (DSC) diagram. Furthermore, the Zeta crystalline form of bilastine has the DSC diagram shown in FIG. 29.

The data of the structure of the previously defined Zeta crystalline form of bilastine obtained by single-crystal x-ray diffraction corresponds to a pentahydrate and is shown below:

| | |
|---|---|
| Empirical formula | C₂₈ H₄₇ N₃ O₈ |
| Molecular weight | 553.68 |
| Temperature | 100(2) K |
| Wavelength | 0.71073 Å |
| Crystal system | monoclinic |
| Space group | P 21/c |
| Unit cell parameters: | a = 11.4856(5) Å α= 90°. |
| | b = 8.5007(4) Å β= 99.765(2)°. |
| | c = 30.4880(14) Å γ = 90°. |
| Volume | 2933.6(2) Å3 |
| Z | 4 |
| Density (calculated) | 1.254 Mg/m3 |
| Absorption coefficient | 0.091 mm-1 |
| F(000) | 1200 |
| Crystal size | 0.196mm x 0.250mm x 0.389mm |
| Theta range | 2.430 to 31.465°. |
| Index ranges | -16<=h<=16. -12<=k<=12. -44<=l<=44 |
| Reflections collected | 100284 |
| Independent reflections | 9660 [R(int) = 0.0553] |
| Completeness to theta= 25.242° | 99.9 % |
| Refinement method | Full-matrix least-squares on F2 |
| Data / restraints / parameters | 9660 / 15 / 385 |
| Goodness-of-fit on F2 | 1.040 |
| Final R indices [I>2sigma(I)]. | R1 = 0.0454. wR2 = 0.1043 |
| R Indices | R1 = 0.0700. wR2 = 0.1146 |
| Extinction coefficient | n/a |
| Largest difference peak and hole | 0.432 and -0.287 e.Å-3 |

Throughout the description and claims the word "comprise" and variations of the word, are not intended to exclude other technical features, additives, components, or steps. Furthermore, the word "comprise" encompasses the case of "consisting of". Additional objects, advantages and features of the invention will become apparent to those skilled in the art upon examination of the description or may be learned by practice of the invention. The following examples and drawings are provided by way of illustration, and they are not intended to be limiting of the present invention. Reference signs related to drawings and placed in parentheses in a claim, are solely for attempting to increase the intelligibility of the claim, and shall not be construed as limiting the scope of the claim. Furthermore, the present invention covers all possible combinations of particular and preferred embodiments described herein.

### Brief description of the drawings

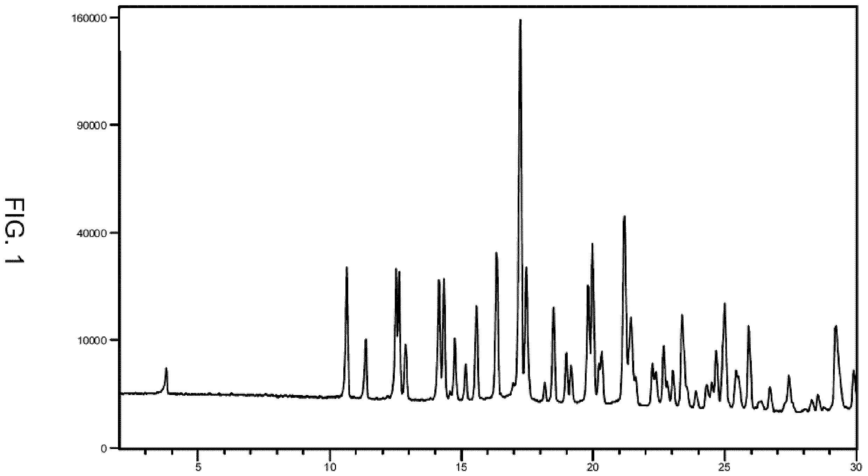

FIG. 1 shows the pattern of X-ray powder diffraction (intensity (counts) vs. angle 2-theta (°)) of crystalline form 1 of bilastine known in the state of the art.

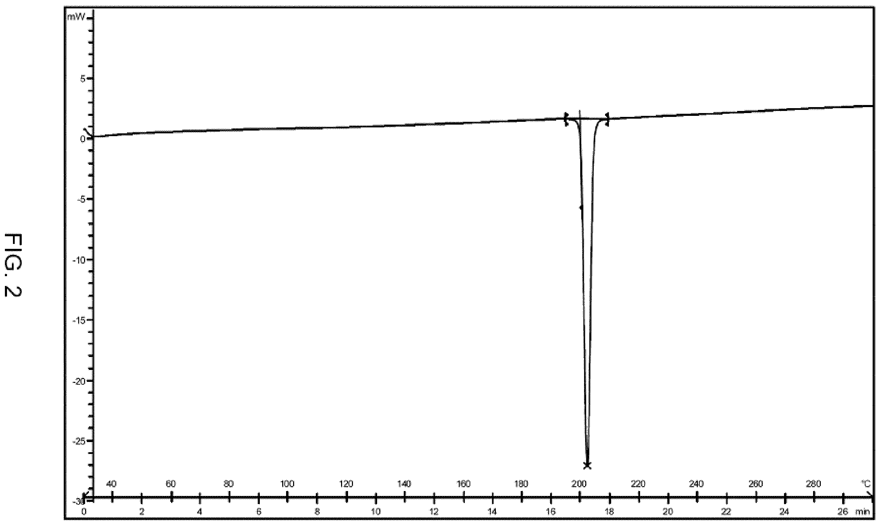

FIG. 2 shows the DSC curve of crystalline form 1 of bilastine known in the state of the art.

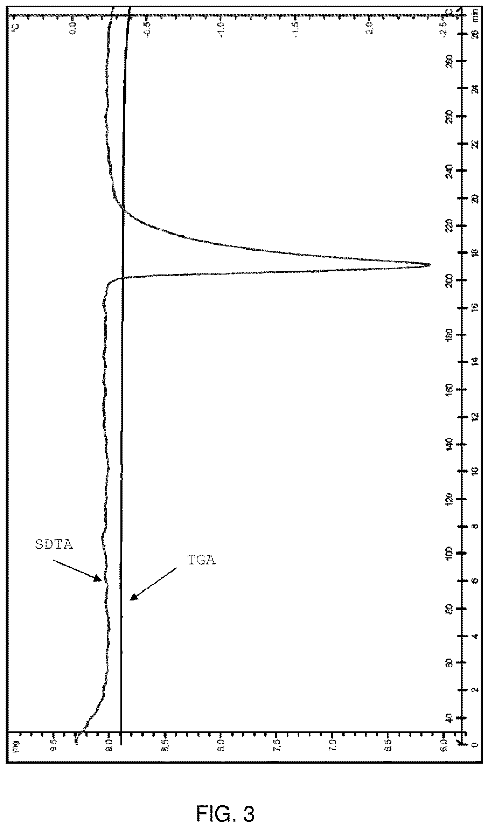

FIG. 3 shows the TGA diagram of crystalline form 1 of bilastine known in the state of the art.

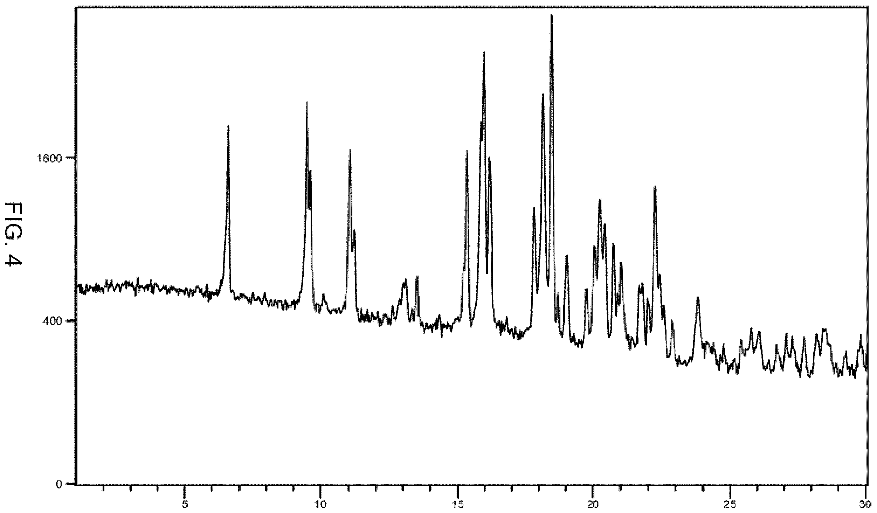

FIG. 4 shows the pattern of X-ray powder diffraction (intensity (counts) vs. angle 2-theta (°)) of crystalline form 2 of bilastine known in the state of the art.

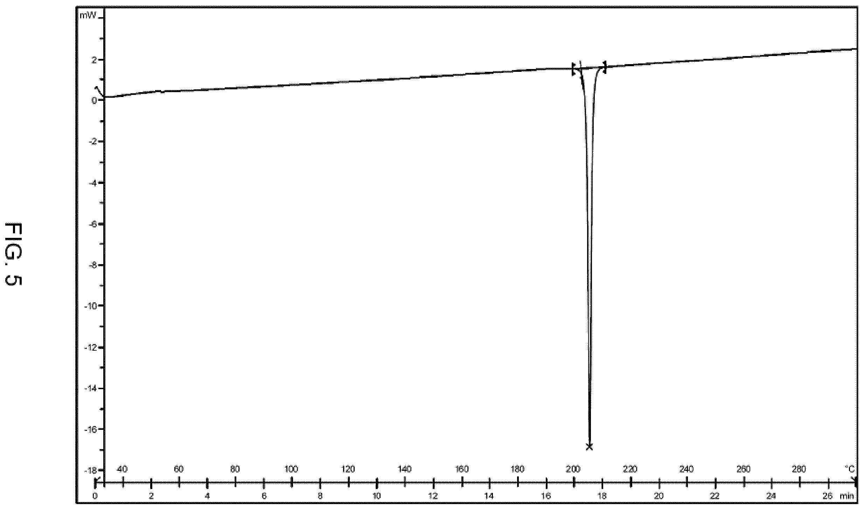

FIG. 5 shows the DSC curve of crystalline form 2 of bilastine known in the state of the art.

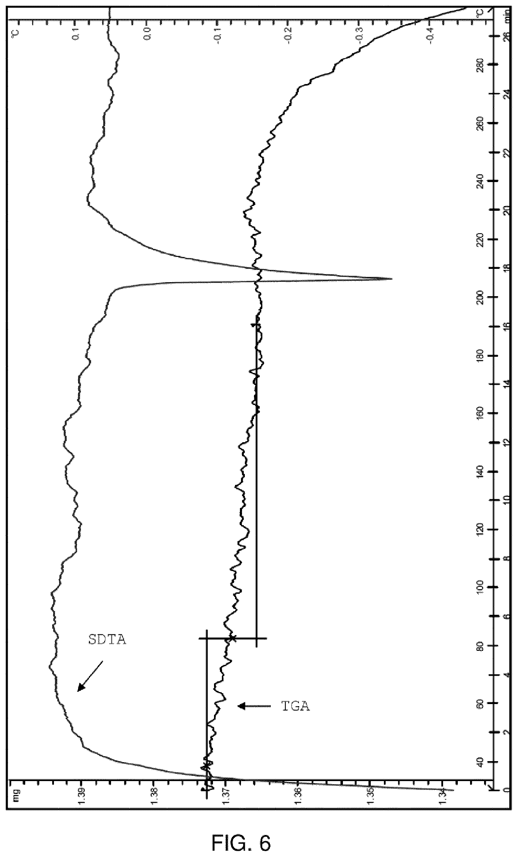

FIG. 6 shows the TGA diagram of crystalline form 2 of bilastine known in the state of the art.

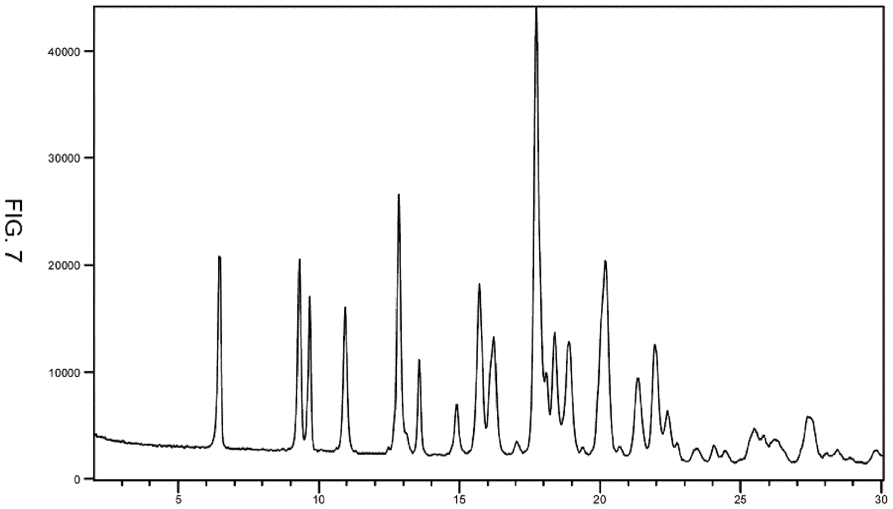

FIG. 7 shows the pattern of X-ray powder diffraction (intensity (counts) vs. angle 2-theta (°)) of crystalline form 3 of bilastine known in the state of the art.

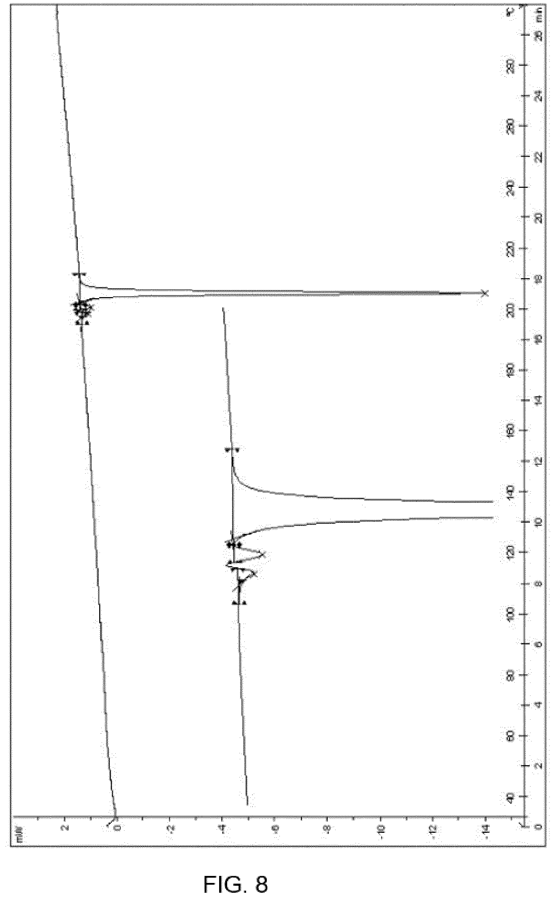

FIG. 8 shows the DSC curve of crystalline form 3 of bilastine known in the state of the art.

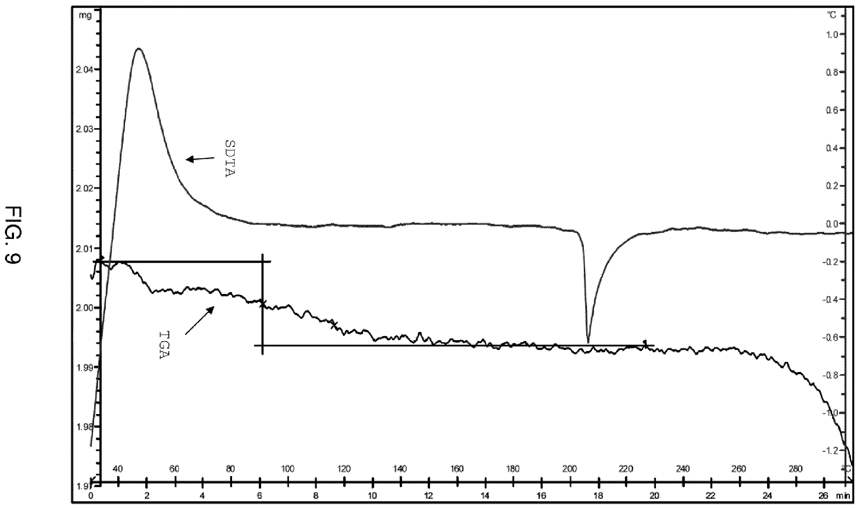

FIG. 9 shows the TGA diagram of crystalline form 3 of bilastine known in the state of the art.
FIG. 10 shows the pattern of X-ray powder diffraction (intensity (counts) vs. angle 2-theta (°)) of the previously defined Alpha crystalline form of bilastine.
FIG. 11 shows the DSC curve of the previously defined Alpha crystalline form of bilastine.
FIG. 12 shows the TGA diagram of the previously defined Alpha crystalline form of bilastine.
FIG. 13 shows the pattern of X-ray powder diffraction (intensity (counts) vs. angle 2-theta (°)) of Beta crystalline form of bilastine.
FIG. 14 shows the DSC curve of the Beta crystalline form of bilastine.
FIG. 15 shows the TGA diagram of the Beta crystalline form of bilastine.
FIG. 16 shows the pattern of X-ray powder diffraction (intensity (counts) vs. angle 2-theta (°)) of Delta crystalline form of bilastine.
FIG. 17 shows the DSC curve of the Delta crystalline form of bilastine.
FIG. 18 shows the TGA diagram of the Delta crystalline form of bilastine.
FIG. 19 shows the pattern of X-ray powder diffraction (intensity (counts) vs. angle 2-theta (°)) of Epsilon crystalline form of bilastine.
FIG. 20 shows the DSC curve of the Epsilon crystalline form of bilastine.
FIG. 21 shows the TGA diagram of the Epsilon crystalline form of bilastine.
FIG. 22 shows the pattern of X-ray powder diffraction (intensity (counts) vs. angle 2-theta (°)) of Gamma A crystalline form of bilastine.
FIG. 23 shows the DSC curve of the Gamma A crystalline form of bilastine.
FIG. 24 shows the TGA diagram of the Gamma A crystalline form of bilastine.
FIG. 25 shows the pattern of X-ray powder diffraction (intensity (counts) vs. angle 2-theta (°)) of Gamma B crystalline form of bilastine.
FIG. 26 shows the DSC curve of the Gamma B crystalline form of bilastine.
FIG. 27 shows the TGA diagram of the Gamma B crystalline form of bilastine.
FIG. 28 shows the pattern of X-ray powder diffraction (intensity (counts) vs. angle 2-theta (°)) of Zeta crystalline form of bilastine.
FIG. 29 shows the DSC curve of the Zeta crystalline form of bilastine.
FIG. 30 shows the pattern of X-ray powder diffraction (intensity (counts) vs. angle 2-theta (°)) of Eta crystalline form of bilastine.
FIG. 31 shows the DSC curve of the Eta crystalline form of bilastine.
FIG. 32 shows the TGA diagram of the Eta crystalline form of bilastine.

### Examples

### General considerations

The X-ray diffractogram has been obtained by using an X-ray diffractometer with Cu Kα radiation (1.5418 Å) in a PANalytical X'Pert PRO MPD powder diffractometer with a radius of 240 millimeters, in a convergent beam configuration with a focusing mirror and transmission geometry with flat samples inserted between low absorbent films. The samples in powder form were inserted between polyester films with a thickness of 3.6 microns and the experimental conditions were as follows:
Cu K radiationα (λ = 1.5418 Å).
Working power: 45 kV - 40 mA.
Incident beam slits that define a beam height of 0.4 millimeters
Incident and diffracted beam slits of 0.02 radian Soller
*Detector PIXcel:* active length = 3.347 °
2θ/θ scans of 2 to 40 °2θ with a step size of 0.026 °2θ and a measurement time of 76 seconds per step.
   The diffractograms obtained show the pattern of X-ray powder diffraction (intensity (counts) vs. angle 2-theta (°)).

The diffractograms of single-crystal X-ray diffraction were obtained by using a D8 Venture single-crystal X-ray diffraction diffractometer (SCXRD) equipped with a multi-layer monochromator and a microfocus Mo (λ = 0.71073 Å). The margins were integrated with Bruker SAINT software by using a SAINT algorithm. The data was corrected for absorption effects using a multi-scan method (SADABS). The structure was resolved and refined by using the Bruker SHELXTL Software Package (cf. George M. Sheldrick. Acta Cryst. (2008). A64. 112-122). a computer program for a full-matrix method of least-squares with SheIXIe Version 4.8.0 C. B. Hübschle. (G. M. Sheldrick and B. Dittrich: a Qt graphical user interface for SHELXL (cf. J. Appl. Cryst. 44. (2011) 1281-1284), a crystal structure refinement program.

The differential scanning calorimetry (DSC) diagrams have been obtained by a Mettler-Toledo DSC-822e calorimeter. The experimental conditions are the following: aluminum crucibles with a volume of 40µL, a dry nitrogen atmosphere with a flow rate of 50 mL/min and a heating rate of 10°C/min. The calorimeter was calibrated with Indium of 99.99% purity. The DSC curves show heat flows (mW) vs. time and temperature. The diagrams express upward the exothermic phenomena (∧EXO).

The diagrams showing thermogravimetric analysis (TGA) have been obtained by a Mettler-Toledo Toledo TGA-851e thermobalance instrument using aluminum crucibles with a volume of 70µl, a dry nitrogen atmosphere with a flow of 50mL/min and a heating rate of 10°C/min. The diagrams simultaneously show the variation in the mass of a sample upon heating it (TGA), as well as the SDTA signal expressed in milligrams (mg) vs. min (minutes) and °C (temperature).

The water content of the Alpha and Eta forms were obtained by volumetric titration according to the Karl Fischer method (KFT) which is expressed in percentage (%) by weight. The Alpha crystalline form was kept at room temperature and at a relative humidity of approximately 67% for 24 hours.

The measurement of the particle size of the crystalline forms 2, Alpha and Eta was obtained by laser diffraction using a Malvern Mastersizer 3000 model particle size analyzer by solid means.

### Comparative example 1. Preparation of the crystalline form 1 of bilastine

### Method 1A

20 mg of bilastine (0.043 mmol) were dissolved in 1.0 mL of ethanol at room temperature and the solvent was left to evaporate for 24 hours. After this time, needle-like crystals were formed and filtered. The analysis of X-ray diffraction resulted in the diffractogram shown in FIG. 1.

### Method 1B

20 mg of bilastine (0.043 mmol) were dissolved in 0.5 mL of methanol at 60°C and the solution was slowly cooled until room temperature was reached. After 24 hours, the solid crystallized and was subsequently filtered and dried in a vacuum. The analysis of X-ray diffraction resulted in the diffractogram shown in FIG. 1.

### Method 1C

20 mg of bilastine (0.043 mmol) were dissolved in 1.8 mL of ethanol at 70°C and the solution was slowly cooled until room temperature was reached. After 24 hours, the solid crystallized and was subsequently filtered and dried in a vacuum. The analysis of X-ray diffraction resulted in the diffractogram shown in FIG. 1.

The water content of two different samples of crystalline form 1 obtained by this method was analyzed. The water content of said samples was 7.9% and 8.1% respectively.

### Method 1D

20 mg of bilastine (0.043 mmol) were dissolved in 2.0 mL of isopropyl alcohol at 82°C and the solution was slowly cooled until room temperature was reached. After 24 hours, the solid crystallized and was subsequently filtered and dried in a vacuum. The analysis of X-ray diffraction resulted in the diffractogram shown in FIG. 1.

The analysis of the unit cell parameters of single-crystal crystalline elucidation confirmed that the crystalline form 1 obtained by the methods described in the comparative example 1 coincided with the crystalline form 1 described in the European patent EP1505066B1 of the state of the art.

### Comparative example 2. Preparation of the crystalline form 2 of bilastine

### Method 2A

20 mg of the Beta crystalline form of bilastine (0.043 mmol) were added to aluminum crucibles of 70 µl and heated inside a TGA instrument in a nitrogen atmosphere, wherein the temperature was increased from 30°C to 198°C, an increase carried out at a rate of 10°C per minute. It was kept at 198°C for 2 minutes and subsequently cooled at room temperature. The analysis of X-ray diffraction resulted in the diffractogram shown in FIG. 4 and the TGA analysis shows a weight loss of 0.5% between 30°C and 191°C.

### Method 2B

The crystalline form 2 was prepared by reproducing the method 2A described above with 100 g of the Beta crystalline form of bilastine, and the product obtained was milled in a Retsch ZM200 model ultra centrifugal mill until obtaining a particle size of d10=4.3 µm; d50=27.0 µm and d90=103 µm.

The melting point confirmed that the crystalline form 2 obtained by the methods described in the comparative example 2 (method 2A and 2B) coincided with the crystalline form 2 described in the European patent EP1505066B1 of the state of the art.

### Comparative example 3. Preparation of the crystalline form 3 of bilastine

### Method 3A

20 mg of bilastine (0.043 mmol) were dissolved in 0.2 mL of chloroform (CHCl₃) at 60°C and the solution was slowly cooled only turning off the heat source once room temperature was reached. After 6 days, the solid crystallized and was subsequently filtered and dried in a vacuum. The analysis of X-ray diffraction resulted in the diffractogram shown in FIG. 7.

### Method 3B

20 mg of bilastine (0.043 mmol) were dissolved in 0.2 mL of chloroform (CHCl₃) at 60°C and the solution was slowly cooled, turning off the heat source and removing it from the heat source once room temperature was reached. After 6 days, the solid crystallized and was subsequently filtered and dried in a vacuum. The analysis of X-ray diffraction resulted in the diffractogram shown in FIG. 7.

### Method 3C

1.0 mL of diethyl ether was added to a solution of 100 mg of bilastine (0.216 mmol) in 0.5 mL of chloroform (CHCl₃) at room temperature. It was cooled to 0°C and after two hours, a white solid crystallized. Subsequently, the solid was filtered and dried in a vacuum. The analysis of X-ray diffraction resulted in the diffractogram shown in FIG. 7.

### Method 3D

50 mg of bilastine (0.108 mmol) were suspended in 0.3 mL of chloroform (CHCl₃). The suspension was continuously stirred for 24 hours at room temperature. The solid crystallized and was subsequently filtered and dried in a vacuum. The analysis of X-ray diffraction resulted in the diffractogram shown in FIG. 7.

### Method 3E

100 mg of bilastine (0.216 mmol) were dissolved in 0.65 mL of chloroform (CHCl₃) at 60°C and the solution was slowly cooled until room temperature was reached. The solid precipitated and was subsequently filtered and dried in a vacuum. The analysis of X-ray diffraction resulted in the diffractogram shown in FIG. 7 and the TGA analysis shows a weight loss of 0.7% between 33°C and 226°C.

The melting point confirmed that the crystalline form 3 obtained by the methods described in the comparative example 3 coincided with the crystalline form 3 described in the European patent EP1505066B1 of the state of the art.

### Example 1. Preparation of the previously defined Alpha crystalline form of bilastine

### Method 1A

20 mg of bilastine form 1 (0.043 mmol) were suspended in 2.0 mL of water. The suspension obtained was heated to a temperature of 90°C and was left to temper until room temperature was reached. The suspension was left stirring for 72 hours at room temperature. Subsequently, the solid in suspension was filtered and dried in a vacuum. The analysis of X-ray diffraction resulted in the diffractogram shown in FIG. 10.

### Method 1B

100 mg of bilastine form 1 (0.216 mmol) were suspended in 2.0 mL of water. The suspension obtained was heated to a temperature of 90°C and was left to temper until room temperature was reached. The suspension was left stirring for 72 hours at room temperature. Subsequently, the solid in suspension was filtered and dried in a vacuum. The analysis of X-ray diffraction resulted in the diffractogram shown in FIG. 10.

### Method 1C

100 mg of bilastine form 1 (0.216 mmol) were suspended in 0.6 mL of water. The suspension obtained was left stirring for 72 hours at room temperature. Subsequently, the solid in suspension was filtered and dried in a vacuum. The analysis of X-ray diffraction resulted in the diffractogram shown in FIG. 10.

### Method 1D

250 mg of bilastine, 2.5 L of water and 1.75 L of EtOH 96° were loaded into a reactor and heated under reflux until complete dissolution. The resulting mixture was filtered while hot in a 5 L reactor, previously heated to T=90°C (if after filtration a precipitated is observed, it is heated again until the possible remains of the solid have been dissolved).

Once the entire solid was dissolved, the mixture was cooled to T=68°C and was seeded with 0.25 g of the Alpha form of bilastine. Next, the mixture was cooled to T=20°C at an approximate rate of 0.7°C/min. The precipitated solid was filtered and dried in a vacuum at a temperature of 35°C until the water content was approximately 7% by weight (calculated by the Karl Fischer method (KFT)). 242.93 g of a solid that corresponded to the Alpha form of bilastine with a KF of 7.4% by weight were obtained. This obtained product was milled in a Frewitt Hammerwitt-LAB Valve Witt-80 model hammer mill, obtaining two bilastines with different particle sizes:
Alpha form of bilastine 1D-a: d10=11.4µm; d50=43.7µm and d90=167 µm.
Alpha form of bilastine 1D-b: d10=10.1µm; d50=31.0µm and d90=86.9 µm.

### Method 1E

510 mL of water, 510 g of ice and 25.5 g of potassium hydroxide (KOH) were mixed in a reactor, and 169 g of bilastine were added to this solution. It was slowly heated to dissolve the entire solid, filtered in a 2 L thermostatically-controlled reactor and washed with 340 mL of water. The mixture was heated to T=45°C and a solution of HCl 2N was added until the pH was adjusted to 7.2. During the adjustment of pH, a solid precipitated. After the adjustment of pH, it was kept at T=45°C for 45 minutes. After this time, it was cooled to a temperature between 20-25°C and continuously stirred at this temperature for approximately 17 hours. The obtained solid was centrifuged and washed with 440 mL of water. The solid was drained for 30 min and dried in a vacuum at a T=35°C until the water content was approximately 7% by weight (calculated by the Karl Fischer method (KFT)). The solid obtained corresponded to the Alpha form of bilastine.

### Method 1F

459.25 mL of water, 459.25 g of ice and 16.5 g of potassium hydroxide (KOH) were mixed in a reactor, and 110.16 g of bilastine was added to this solution. Once dissolved, the solid was filtered to a 2 L thermostatically-controlled reactor and washed with 187 mL of water and 368.5 mL of isopropanol. A solution of HCI 2N was added to this mixture at T=23°C until the pH was adjusted to 7.2. During the adjustment of pH, a solid precipitated. After the adjustment of pH, it was continuously stirred at T=20-25°C for 21 hours. The obtained solid was centrifuged and washed with 220 mL of water, drained and dried in a vacuum at a T=35°C until the water content was approximately 7% by weight (calculated by the Karl Fischer method (KFT)). The solid obtained corresponded to the Alpha form of bilastine.

### Example 2. Preparation of the Beta crystalline form of bilastine

20 mg of bilastine form 1 (0.043 mmol) were suspended in 1.0 mL of methanol. The obtained suspension was left stirring at room temperature and after 3 hours, the solid in suspension was filtered and dried in a vacuum. The analysis of X-ray diffraction resulted in the diffractogram shown in FIG. 13.

### Example 3. Preparation of the Delta crystalline form of bilastine

20 mg of bilastine form 1 (0.043 mmol) were dissolved in 2.0 mL of dioxane at a temperature of 70°C. The solution obtained was left to cool in a water-ice bath. After 2 hours, the solution was kept at 4°C for 72 hours. After this time, the precipitated solid was filtered and dried in a vacuum. The analysis of X-ray diffraction resulted in the diffractogram shown in FIG. 16.

### Example 4. Preparation of the Epsilon crystalline form of bilastine

20 mg of bilastine (0.043 mmol) were dissolved in 0.55 mL of dichloromethane at room temperature. Subsequently. 2.0 mL of water were added and the solution was kept at room temperature for one week. After this time, the precipitated solid was filtered and dried in a vacuum. The analysis of X-ray diffraction resulted in the diffractogram shown in FIG. 19.

### Example 5. Preparation of the Gamma A crystalline form of bilastine

50 mg of bilastine (0.106 mmol) were dissolved in 0.2 mL of CHCl₃ at a temperature of 60°C. The solution obtained was left to cool in a water-ice bath. After 2 hours, the solution was kept at 4°C for 72 hours. After this time, the precipitated solid was filtered and dried in a vacuum. The analysis of X-ray diffraction resulted in the diffractogram shown in FIG. 22.

### Example 6. Preparation of the Gamma B crystalline form of bilastine

### Method 6A

50 mg of bilastine (0.106 mmol) were dissolved in 0.2 mL of CHCl₃ at a temperature of 60°C. The solution obtained was left to cool in a water-ice bath. After 2 hours, the solution was kept at 4°C for 12 hours. After this time, the precipitated solid was filtered and dried in a vacuum. The analysis of X-ray diffraction resulted in the diffractogram shown in FIG. 25.

### Method 6B

20 mg of bilastine (0.043 mmol) were dissolved in 0.5 mL of CHCl₃ at a room temperature. The solution obtained was left to cool in a water-ice bath. After 2 hours, the solution was kept at 4°C for 12 days. After this time, the precipitated solid was filtered and dried in a vacuum. The analysis of X-ray diffraction resulted in the diffractogram shown in FIG. 25.

### Example 7. Preparation of the Zeta crystalline form of bilastine

50 mg of bilastine (0.107 mmol) were solubilized in 1.0 mL of a mixture of water:acetonitrile (1:1). The obtained solution was stirred for 20 hours at room temperature until the solid crystallized. Subsequently, the solid was filtered and dried in a vacuum. The analysis of X-ray diffraction resulted in the diffractogram shown in FIG. 28.

### Example 8. Preparation the of Eta crystalline form of bilastine

### Method 8A

The zeta form obtained in example 7 was introduced into a dryer at 40°C and 75% relative humidity for one month. The analysis of X-ray diffraction resulted in the diffractogram shown in FIG. 30.

### Method 8B

100 g of bilastine, 587 mL of acetonitrile and 293 mL of water were loaded into a reactor. The mixture was heated to T=55-60°C until complete dissolution of the solid. The solution was filtered to another reactor with mechanical stirring, previously heated at T=50-55°C, and subsequently washed with a mixture of 100 mL of water and 200 mL of acetonitrile at the same temperature, and added to the previously filtered mixture.

The filtered solution mixture was cooled to T=40°C, ensuring that the solid would not precipitate during this time, and was seeded with 1.3 g of the Eta form of bilastine. Next, it was kept at T=35-40°C for 40-45 min, and lastly, cooled to a temperature of 5°C and was continuously stirred at this temperature for 2 hours.

The precipitated solid was centrifuged and washed with 200 mL of cold water, drained and dried in a vacuum at T=35°C until the water content was between 3.5-4.0% by weight (calculated by the Karl Fischer method (KFT)). 85.60 g of the Eta form of bilastine was obtained with a particle size of d10=4.5 µm; d50=15.8 µm and d90=37.9 µm.

### Method 8C

25 g of the Alpha form of bilastine and 250 mL of water were introduced into a 500 mL Erlenmeyer flask equipped with magnetic stirring. The suspension was continuously stirred between 20 and 25°C for 4 days.

The obtained solid was filtered and washed with 50 mL of water. It was dried in a vacuum oven at T=35°C until the water content was between 3.5-4.0% by weight (calculated by the Karl Fischer method (KFT)). 21.07 g of the Eta form of bilastine was obtained.

### Example 9. Stability study

The stability study was carried out with the crystalline forms of bilastine already known in the state of the art (crystalline forms 1, 2 and 3) and the crystalline forms of bilastine described in the present invention. The crystalline forms used in the stability study were prepared following the methods described in the present invention.

The stability study comprises storing each of the crystalline forms of bilastine separately and under different conditions of time, temperature and relative humidity. The conditions of the stability study were the following

The results obtained from said stability study are described in Table 1:

**Table 1**

| Crystalline form | Conditions | | | | | |
|---|---|---|---|---|---|---|
| | 1 month | | 2 months | | 3 months and 6 months | |
| | 25°C/ 60% RH | 40°C/ 75% RH | 25°C/ 60% RH | 40°C/ 75% RH | 25°C/ 60% RH | 40°C/ 75% RH |
| 1 | 1 | 1 | 1 | 1 | 1 | 1 |
| 2 | 2 | 2 | 2 | 2 | 2 | 2 |
| 3 | 3+1 | 3 + 1 | 3+1 | 1 + 3 | - | - |
| Alpha | Alpha | Alpha | Alpha | Alpha | Alpha | Alpha |
| Beta | Beta + 3 | - | - | - | - | - |
| Gamma A | 2+3+ other crystalline forms | 3 + 1 | - | - | - | - |
| Gamma B | Gamma B + 3 | 1+3+ Alpha | - | | - | |
| Delta | 1 + Epsilon | 1 | 1 | 1 | - | - |
| Epsilon | 1 + Eta | 1 + Eta | - | - | - | - |
| Zeta | Zeta | Eta | - | - | - | - |
| Eta | Eta | Eta | Eta | Eta | Eta | Eta |

| | | | | | | |
|---|---|---|---|---|---|---|
| RH represents relative humidity | | | | | | |

From the results of Table 1, it can be concluded that the Alpha and Eta crystalline forms of bilastine are stable under the conditions described until at least 6 months. Therefore, the Alpha and Eta crystalline forms of bilastine are stable and suitable for preparing a pharmaceutical composition of bilastine.

### Example 10. Formulations

### Composition

The quantitative compositions of tablets that comprise the previously defined Alpha crystalline form of bilastine of Examples 1 D-a and 1 D-b or the Eta crystalline form of bilastine of Example 8b of the present invention, as well as the crystalline form 2 of bilastine of Comparative example 2B are described below.

The amounts of the ingredients expressed in milligrams per table are described below in Table 2.

**Table 2**

| **Ingredients** | **Composition** | | | |
|---|---|---|---|---|
| | **10A** | **10B** | **10C** | **10D** |
| **Crystalline form of bilastine** | **Alpha Ex. 1D-a** | **Alpha ex. 1D-b** | **Eta ex. 8B** | **2 ex. comp.2B** |
| Bilastine(*) | 21.45 | 21.46 | 20.76 | 20.27 |
| Microcrystalline cellulose | 96.68 | 96.67 | 97.37 | 97.86 |
| Magnesium stearate | 1.25 | 1.25 | 1.25 | 1.25 |
| Sodium carboxymethyl starch | 5.00 | 5.00 | 5.00 | 5.00 |
| Colloidal anhydrous silica | 0.63 | 0.63 | 0.63 | 0.63 |

| | | | | |
|---|---|---|---|---|
| (*) Equivalent to 20 mg of anhydrous bilastine; amount calculated by % water KF | | | | |

### Preparation method

On one hand, the tablet 10A was obtained by wet granulation; while the tablets 10B, 10C and 10D were obtained by direct compression. The methods used in each case are described below.

### Wet granulation

Microcrystalline cellulose, sodium carboxymethyl starch and bilastine were added to the mixer in the amounts specified in Table 2. The resulting mixture was homogenized in a turbula-type mixer and kneaded with 135 g of purified water in a planetary stirrer and dried for 2 hours at 50°C. The granules obtained were mixed with the amount of colloidal anhydrous silica specified in Table 2 in a turbula-type mixer, and then were mixed with the amount of magnesium stearate specified in Table 2. Finally, the tablets were prepared by compressing the mixture obtained in an eccentric compressing machine.

### Direct compression

Microcrystalline cellulose, sodium carboxymethyl starch, bilastine and colloidal anhydrous silica were added to the mixer in the amounts specified in Table 2. The resulting mixture was homogenized in a turbula-type mixer, and then the amount of magnesium stearate specified in Table 2 was added and mixed in a turbula-type mixer. Finally, the tablets were prepared by compressing the mixture obtained in an eccentric compressing machine.

In all the tablets prepared in the present invention, the crystalline form of bilastine used as a starting ingredient was maintained. The tablets obtained were conditioned in the shape of Alu/Alu, PVC/Alu and PVC/PVDC(90 g/m2)/Alu blisters.

## Claims

1. A hydrated Eta crystalline form of bilastine **characterized in that** it has an X-ray diffractogram that comprises characteristic peaks at 8.4; 9.6; 12.2; 13.2; 15.1; and 19.2 ± 0.2 degrees 2 theta measured with an X-ray diffractometer with Cu Kα radiation (1.5418 Å).

2. The crystalline form of bilastine according to claim 1, wherein the X-ray diffractogram further comprises characteristic peaks at 19.7; 20.3; 21.5; and 23.4 ± 0.2 degrees 2 theta.

3. The crystalline form of bilastine according to any of claims 1-2, wherein the X-ray diffractogram further comprises characteristic peaks at 14.0; 16.8; 17.5; 18.2 and 25.5 ± 0.2 degrees 2 theta.

4. The crystalline form of bilastine according to any of claims 1-3, **characterized in that** it has a DSC that comprises a first broad endothermic phenomenon with a peak at 137°C and an associated heat of 35.4 J/g followed by a second endothermic phenomenon at 198°C with an associated heat of 13.4 J/g overlapped with an exothermic phenomenon with a peak at 200°C and an associated heat of 14.0 J/g and followed by a third endothermic phenomenon at 204°C with an associated heat of 101.3 J/g.

5. The crystalline form of bilastine according to any of claims 1-4, **characterized by** a thermogravimetric analysis that comprises a weight loss of 4.0% from 30°C to 120°C.

6. A preparation method of the Eta crystalline form of bilastine defined in any of claims 1-5, **characterized in that** it comprises dispersing Alpha crystalline form of bilastine **characterized in that** it has an X-ray diffractogram that comprises characteristic peaks at 8.7; 11.6; 13.4; 13.8, 14.0 and 17.7 ± 0.2 degrees 2 theta measured with an X-ray diffractometer with Cu Kα radiation (1.5418 Å) or a mixture of Alpha and Eta crystalline form of bilastine in water during the period of time needed for the transformation into the Eta crystalline form of bilastine to take place.

7. The preparation method of the Eta crystalline form of bilastine defined in any of claims 1-5, **characterized in that** it comprises the following steps:
i) dissolve bilastine in a mixture of water and acetonitrile at a temperature comprised from 40°C to 70°C;
ii) cool the solution obtained in step i) to a temperature comprised from 25°C to 50°C;
iii) seed the solution obtained in step ii) with the Eta crystalline form and cool the resulting solution to a temperature comprised from 0°C to 30°C during the period of time needed for the crystallization to take place; and
iv) dry the crystalline form obtained in step iii) under reduced pressure at a temperature comprised from 25°C to 40°C until the water content is comprised from 3.5% to 4% by weight calculated by the Karl Fischer method.

8. The preparation method of the Eta crystalline form of bilastine defined in claim 7, **characterized in that** in step iv) it is carried out under reduced pressure at a temperature comprised from 30°C to 35°C.

9. The preparation method of the Eta crystalline form of bilastine defined in any of claims 1-5, **characterized in that** it comprises maintaining Zeta crystalline form of bilastine **characterized in that** it has an X-ray diffractogram that comprises characteristic peaks at 7.76, 8.93, 10.45, 10.63, 11.69, 12.96, 13.60, 14.65, 15.07, 15.58, 16.28, 16.53, 17.54, 18.04, 18.25, 18.61, 19.54, 20.38, 20.69, 21.01, 21.37, 21.73, 21.99, 22.37, 22.85, 23.10, 23.25, 23.54, 24.18, 24.37 and 25.12 ± 0.2 degrees 2 theta measured with an X-ray diffractometer with Cu Kα radiation (1.5418 Å) at a temperature comprised from 20°C to 50°C during the period of time needed for the transformation into the Eta crystalline form of bilastine to take place.

10. The preparation method of the Eta crystalline form of bilastine defined in claim 9, **characterized in that** it comprises maintaining the Zeta crystalline form at a temperature comprised from 25°C to 40°C.

11. The preparation method of the Eta crystalline form of bilastine defined in any of claims 9-10, **characterized in that** it comprises maintaining the Zeta crystalline form of bilastine at a temperature comprised from 30°C to 35°C until the water content is comprised from 3.5% to 4% by weight calculated by the Karl Fischer method.

12. The preparation method of the Eta crystalline form of bilastine defined in any of claims 1-5, **characterized in that** it comprises maintaining the Zeta crystalline form of bilastine as defined in claim 9 at a temperature comprised from 35°C to 45°C and at a relative humidity comprised from 65% to 80% during the period of time needed for the transformation into the Eta crystalline form of bilastine to take place.

13. The preparation method of the Eta crystalline form of bilastine defined in claim 12, **characterized in that** it comprises maintaining the Zeta crystalline form of bilastine as defined in claim 9 during a period of time longer than 2 weeks.

14. A pharmaceutical composition that comprises a therapeutically effective amount of the Eta crystalline form of bilastine defined in any of claims 1-5 along with pharmaceutically acceptable excipients or carriers.

15. A use of the Eta crystalline form of bilastine defined in any of claims 1-5, for the manufacture of a medicament to treat histamine-mediated disease processes and allergic reactions.

16. The use according to claim 15, wherein the treatment of histamine-mediated disease processes and allergic reactions is selected from the group consisting of the symptomatic treatment of seasonal allergic rhinoconjunctivitis, the symptomatic treatment of perennial allergic rhinoconjunctivitis and the treatment of urticaria.

## Patentansprüche

1. Eine hydratisierte kristalline Form Eta von Bilastin, **dadurch gekennzeichnet, dass** sie ein Röntgen-Diffraktogramm hat, das charakteristische Peaks bei 8,4; 9,6; 12,2; 13,2; 15,1; und 19,2 ± 0,2 Grad 2-Theta, gemessen mit einem Röntgen-Diffraktometer mit Cu-Kα-Strahlung (1,5418 Å), umfasst.

2. Die kristalline Form von Bilastin nach Anspruch 1, wobei das Röntgen-Diffraktogramm ferner charakteristische Peaks bei 19,7; 20,3; 21,5; und 23,4 ± 0,2 Grad 2-Theta umfasst.

3. Die kristalline Form von Bilastin nach einem der Ansprüche 1-2, wobei das Röntgen-Diffraktogramm ferner charakteristische Peaks bei 14,0; 16,8; 17,5; 18,2 und 25,5 ± 0,2 Grad 2-Theta umfasst.

4. Die kristalline Form von Bilastin nach einem der Ansprüche 1-3, **dadurch gekennzeichnet, dass** sie eine DSC hat, die ein erstes breites endothermisches Phänomen mit einem Peak bei 137 °C und eine verknüpfte Wärmemenge von 35,4 J/g, gefolgt von einem zweiten endothermischen Phänomen bei 198 °C mit einer verknüpften Wärmemenge von 13,4 J/g, überlappt mit einem exothermischen Phänomen mit einem Peak bei 200 °C und einer verknüpften Wärmemenge von 14,0 J/g und gefolgt von einem dritten endothermischen Phänomen bei 204 °C mit einer verknüpften Wärmemenge von 101,3 J/g umfasst.

5. Die kristalline Form von Bilastin nach einem der Ansprüche 1-4, **gekennzeichnet durch** eine thermogravimetrische Analyse, die einen Gewichtsverlust von 4,0% von 30 °C bis 120 °C umfasst.

6. Ein Herstellungsverfahren der kristallinen Form Eta von Bilastin, die in einem der Ansprüche 1-5 definiert ist, **dadurch gekennzeichnet, dass** es das Dispergieren kristalliner Form Alpha von Bilastin, **dadurch gekennzeichnet, dass** sie ein Röntgen-Diffraktogramm hat, das charakteristische Peaks bei 8,7; 11,6; 13,4; 13,8, 14,0 und 17,7 ± 0,2 Grad 2-Theta, gemessen mit einem Röntgen-Diffraktometer mit Cu-Kα-Strahlung (1,5418 Å) umfasst, oder einer Mischung aus kristalliner Form Alpha und Eta von Bilastin in Wasser während des erforderlichen Zeitraums für die Vervollständigung der Umwandlung in die kristalline Form Eta von Bilastin umfasst.

7. Das Herstellungsverfahren der kristallinen Form Eta von Bilastin, die in einem der Ansprüche 1-5 definiert ist, **dadurch gekennzeichnet, dass** es die folgenden Schritte umfasst:
i) Lösen von Bilastin in einer Mischung aus Wasser und Acetonitril bei einer Temperatur, die von 40 °C bis 70 °C reicht;
ii) Abkühlen der in Schritt i) erhaltenen Lösung auf eine Temperatur, die von 25 °C bis 50 °C reicht;
iii) Säen der in Schritt ii) erhaltenen Lösung mit der kristallinen Form Eta und abkühlen der erhaltenen Lösung auf eine Temperatur, die von 0 °C bis 30 °C reicht, während des erforderlichen Zeitraums für die Vervollständigung der Kristallisation; und
iv) Trocknen der in Schritt iii) erhaltenen kristallinen Form unter vermindertem Druck bei einer Temperatur, die von 25 °C bis 40 °C reicht, bis der Wassergehalt von 3,5 Gew.-% bis 4 Gew.-%, berechnet durch das Karl-Fischer-Verfahren, reicht.

8. Das Herstellungsverfahren der kristallinen Form Eta von Bilastin, das in Anspruch 7 definiert ist, **dadurch gekennzeichnet, dass** es in dem Schritt iv) unter vermindertem Druck bei einer Temperatur reichend von 30 °C bis 35 °C durchgeführt wird.

9. Das Herstellungsverfahren der kristallinen Form Eta von Bilastin, die in einem der Ansprüche 1-5 definiert ist, **dadurch gekennzeichnet, dass** es das Behalten von kristalliner Form Zeta von Bilastin umfasst, **dadurch gekennzeichnet, dass** sie ein Röntgen-Diffraktogramm umfasst, das charakteristische Peaks bei 7,76, 8,93, 10,45, 10,63, 11,69, 12,96, 13,60, 14,65, 15,07, 15,58, 16,28, 16,53, 17,54, 18,04, 18,25, 18,61, 19,54, 20,38, 20,69, 21,01, 21,37, 21,73, 21,99, 22,37, 22,85, 23,10, 23,25, 23,54, 24,18, 24,37 und 25,12 ± 0,2 Grad 2-Theta, gemessen mit einem Röntgen-Diffraktometer mit Cu-Ka-Strahlung (1,5418 Å) bei einer Temperatur reichend von 20 °C bis 50 °C während des erforderlichen Zeitraums für die Vervollständigung der Umwandlung in die kristalline Form Eta von Bilastin, umfasst.

10. Das Herstellungsverfahren der kristallinen Form Eta von Bilastin, die in Anspruch 9 definiert ist, **dadurch gekennzeichnet, dass** es das Behalten der kristallinen Form Zeta bei einer Temperatur reichend von 25 °C bis 40 °C umfasst.

11. Das Herstellungsverfahren der kristallinen Form Eta von Bilastin, die in einem der Ansprüche 9-10 definiert ist, **dadurch gekennzeichnet, dass** es das Behalten der kristallinen Form Zeta von Bilastin bei einer Temperatur reichend von 30 °C bis 35 °C bis der Wassergehalt von 3,5 Gew.-% bis 4 Gew.-% reicht, berechnet durch das Karl-Fischer-Verfahren, umfasst.

12. Das Herstellungsverfahren der kristallinen Form Eta von Bilastin, die in einem der Ansprüche 1-5 definiert ist, **dadurch gekennzeichnet, dass** es das Behalten der kristallinen Form Zeta von Bilastin wie in Anspruch 9 definiert bei einer Temperatur reichend von 35 °C bis 45 °C und einer relativen Luftfeuchtigkeit reichend von 65% bis 80% während des erforderlichen Zeitraums für die Vervollständigung der Umwandlung in die kristalline Form Eta von Bilastin umfasst.

13. Das Herstellungsverfahren der kristallinen Form Eta von Bilastin, die in Anspruch 12 definiert ist, **dadurch gekennzeichnet, dass** es das Behalten der kristallinen Form Zeta von Bilastin wie in Anspruch 9 definiert während eines Zeitraums von mehr als 2 Wochen umfasst.

14. Eine pharmazeutische Zusammensetzung, welche eine therapeutisch wirksame Menge der kristallinen Form Eta von Bilastin wie in einem der Ansprüche 1-5 definiert, zusammen mit pharmazeutisch akzeptablen Hilfsstoffen oder Trägerstoffen, umfasst.

15. Eine Verwendung der kristallinen Form Eta von Bilastin, die in einem der Ansprüche 1-5 definiert ist, für die Herstellung von einem Arzneimittel zur Behandlung von durch Histamin vermittelten Krankheitsprozessen und allergischen Reaktionen.

16. Die Verwendung nach Anspruch 15, wobei die Behandlung von durch Histamin vermittelten Krankheitsprozessen und allergischen Reaktionen ausgewählt ist aus der Gruppe bestehend aus der symptomatischen Behandlung von jahreszeitlich bedingter allergischer Rhinokonjunktivitis, der symptomatischen Behandlung von ganzjähriger allergischer Rhinokonjunktivitis und der Behandlung von Urtikaria.

## Revendications

1. Une forme cristalline Êta hydratée de bilastine **caractérisée en ce qu'**elle a un diffractogramme de rayons X qui comprend des pics caractéristiques à 8,4 ; 9,6 ; 12,2 ; 13,2 ; 15,1 ; et 19,2 ± 0,2 dégrées 2 thêta, mesuré avec un diffractomètre de rayons X avec du rayonnement de Cu Kα (1,5418 Å).

2. La forme cristalline de bilastine selon la revendication 1, dans laquelle le diffractogramme de rayons X comprend en outre des pics caractéristiques à 19,7 ; 20,3 ; 21,5 ; et 23,4 ± 0,2 dégrées 2 thêta.

3. La forme cristalline de bilastine selon l'une quelconque des revendications 1-2, dans laquelle le diffractogramme de rayons X comprend en outre des pics caractéristiques à 14,0 ; 16,8 ; 17,5 ; 18,2 et 25,5 ± 0,2 dégrées 2 thêta.

4. La forme cristalline de bilastine selon l'une quelconque des revendications 1-3, **caractérisée en ce qu'**elle a une DSC qui comprend un premier phénomène endothermique large avec un pic à 137 °C et une chaleur associée de 35,4 J/g suivi par un deuxième phénomène endothermique à 198 °C avec une chaleur associée de 13,4 J/g chevauché avec un phénomène exothermique avec un pic à 200 °C et une chaleur associée de 14,0 J/g et suivi par un troisième phénomène endothermique à 204 °C avec une chaleur associée de 101,3 J/g.

5. La forme cristalline de bilastine selon l'une quelconque des revendications 1-4, **caractérisée par** une analyse thermogravimétrique qui comprend une perte de masse de 4,0 % de 30 °C à 120 °C.

6. Un procédé de préparation de la forme cristalline Êta de bilastine définie dans l'une quelconque des revendications 1-5, **caractérisé en ce qu'**il comprend disperser de la forme cristalline Alpha de bilastine **caractérisée en ce qu'**elle a un diffractogramme de rayons X qui comprend des pics caractéristiques à 8,7 ; 11,6 ; 13,4 ; 13,8, 14,0 et 17,7 ± 0,2 dégrées 2 thêta, mesuré avec un diffractomètre de rayons X avec du rayonnement de Cu Kα (1,5418 Å), ou un mélange de forme cristalline Alpha et Êta de bilastine en eau pendant la période de temps nécessaire pour que la transformation en la forme cristalline Êta de bilastine ait lieu.

7. Le procédé de préparation de la forme cristalline Êta de bilastine définie dans l'une quelconque des revendications 1-5, **caractérisé en ce qu'**il comprend les étapes suivantes:
i) dissoudre de la bilastine dans un mélange d'eau et acétonitrile à une température comprise de 40 °C à 70 °C ;
ii) refroidir la solution obtenue dans l'étape i) à une température comprise de 25 °C à 50 °C ;
iii) ensemencer la solution obtenue dans l'étape ii) avec la forme cristalline Êta et refroidir la solution résultante à une température comprise de 0 °C à 30 °C pendant la période de temps nécessaire pour que cristallisation ait lieu ; et
iv) sécher la forme cristalline obtenue dans l'étape iii) sous pression réduite à une température comprise de 25 °C à 40 °C jusqu'à ce que la teneur en eau soit comprise de 3,5 % à 4 % en poids calculée par la méthode de Karl Fischer.

8. Le procédé de préparation de la forme cristalline Êta de bilastine définie dans la revendication 7, **caractérisé en ce que** dans l'étape iv) il est effectué sous pression réduite à une température comprise de 30 °C à 35 °C.

9. Le procédé de préparation de la forme cristalline Êta de bilastine définie dans l'une quelconque des revendications 1-5, **caractérisé en ce qu'**il comprend maintenir de la forme cristalline Zêta de bilastine **caractérisée en ce qu'**elle a un diffractogramme de rayons X qui comprend des pics caractéristiques à 7,76, 8,93, 10,45, 10,63, 11,69, 12,96, 13,60, 14,65, 15,07, 15,58, 16,28, 16,53, 17,54, 18,04, 18,25, 18,61, 19,54, 20,38, 20,69, 21,01, 21,37, 21,73, 21,99, 22,37, 22,85, 23,10, 23,25, 23,54, 24,18, 24,37 et 25,12 ± 0,2 dégrées 2 thêta, mesuré avec un diffractomètre de rayons X avec du rayonnement de Cu Kα (1,5418 Å) à une température comprise de 20 °C à 50 °C pendant la période de temps nécessaire pour que la transformation en la forme cristalline Êta de bilastine ait lieu.

10. Le procédé de préparation de la forme cristalline Êta de bilastine définie dans la revendication 9, **caractérisé en ce qu'**il comprend maintenir la forme cristalline Zêta à une température comprise de 25 °C à 40 °C.

11. Le procédé de préparation de la forme cristalline Êta de bilastine défini dans l'une quelconque des revendications 9-10, **caractérisé en ce qu'**il comprend maintenir la forme cristalline Zêta de bilastine à une température comprise de 30 °C à 35 °C jusqu'à ce que la teneur en eau soit comprise de 3,5 % à 4 % en poids calculée par la méthode de Karl Fischer.

12. Le procédé de préparation de la forme cristalline Êta de bilastine définie dans l'une quelconque des revendications 1-5, **caractérisé en ce qu'**il comprend maintenir la forme cristalline Zêta de bilastine telle que définie dans la revendication 9 à une température comprise de 35 °C à 45 °C et à une humidité relative comprise de 65 % à 80 % pendant la période de temps nécessaire pour que la transformation en la forme cristalline Êta de bilastine ait lieu.

13. Le procédé de préparation de la forme cristalline Êta de bilastine défini dans la revendication 12, **caractérisé en ce qu'**il comprend maintenir la forme cristalline Zêta de bilastine telle que définie dans la revendication 9 pendant une période de temps supérieure à 2 semaines.

14. Une composition pharmaceutique qui comprend une quantité thérapeutiquement efficace de la forme cristalline Êta de bilastine définie dans l'une quelconque des revendications 1-5, avec des excipients ou véhicules pharmaceutiquement acceptables.

15. Une utilisation de la forme cristalline Êta de bilastine définie dans l'une quelconque des revendications 1-5, pour la fabrication d'un médicament pour traiter des processus morbides et des réactions allergiques à médiation d'histamine.

16. L'utilisation selon la revendication 15, dans laquelle le traitement de processus morbides et de réactions allergiques à médiation d'histamine est choisi dans le groupe constitué du traitement symptomatique de la rhinoconjonctivite allergique saisonnière, le traitement symptomatique de la rhinoconjonctivite allergique pérenne et du traitement de l'urticaire.
